# EUROPEAN PATENT APPLICATION

(11) **EP 4 807 017 A2**
(43) Date of publication of application: **16.09.2026**
(21) Application number: 26179111.5
(22) Date of filing: 04.03.2024
(51) Int. Cl.: C12Q 1/6855

(54) **HYBRID SSDNA- AND DSDNA-NGS LIBRARY PREPARATION METHODS**

(30) Priority: 07.03.2023 US 202363488898 P; 17.05.2023 US 202363502826 P
(62) Document number(s) of the earlier application(s) in accordance with Art. 76 EPC: 24717408.9 / 4 677 113
(71) Applicant: Guardant Health, Inc., Palo Alto, CA 94304 (US)
(72) Inventor: KENNEDY, Andrew, Palo Alto, 94304 (US)
(74) Representative: Carpmaels & Ransford LLP

(57) **Abstract**

Methods and systems for hybrid library preparation to improve molecular recovery, provide DNA molecule topology, and/or enable novel multiomic workflows. The methods can improve identification of tumor specific biomarkers which can inform therapy selection.

## Description

### CROSS REFERENCE TO RELATED APPLICATIONS

This application claims the benefit of U.S. Provisional Application No. 63/488,898, filed March 7, 2023 and U.S. Provisional Application No. 63/502,826, filed May 17, 2023.

### FIELD OF THE INVENTION

Described herein are methods and compositions related to detection of nucleic acids and preparations for sample preservation, enrichment, preservation and sequencing.

### BACKGROUND

DNA methylation detection by massively parallel sequencing is a promising methodology for sensitive detection of cancer presence in liquid biopsy, applicable to screening and early detection. Gold standard single-site methylation sequencing, such as bisulfite sequencing provides high resolution of methylation states and changes in cell-free DNA (cfDNA) molecules, but has high molecular losses that limit analytical and clinical sensitivity. This is due to the fact that bisulfite is a harsh chemical treatment that degrades DNA non-specifically in the cytosine deamination reaction that is used to resolve methylated and non-methylated cytosine bases. Improved library prep methodologies are needed to boost molecular recovery in bisulfite sequencing.

Next generation sequencing (NGS) library preparation methodologies exist that either act on double-stranded DNA (dsDNA) or single-stranded DNA (ssDNA) molecules as substrates, double stranded DNA library prep (dsDNA-LP) and single stranded DNA library prep (ssDNA-LP), respectively. One or the other is used based on which is more appropriate for a given situation as they have different benefits and suitability. Methods are needed which combine aspects of both workflows in 'hybrid' library prep workflows can improve molecular recovery, provide information on DNA molecule topology, and enable novel multiomic workflows.

### SUMMARY OF THE INVENTION

The present disclosure provides methods and systems to leverage the features of both dsDNA and ssDNA library preparation types to boost molecular recovery, such as with bisulfite sequencing. Such methods that combine aspects of both workflows in a 'hybrid' library preparation can improve molecular recovery, provide information on DNA molecule topology, and enable novel multiomic workflows. High molecular recovery is of paramount importance in early cancer detection and minimal residual disease (MRD) monitoring. Integration of genomic, epigenomic, and molecular topology information can further amplify disease-specific signals to monitor treatment response, resistance and/or identify markers of disease initiation, progression, and metastasis in cfDNA or tissue. Furthermore, these methods may provide a deeper understanding of the changes in DNA and proteins that cause cancer, allowing the identification of tumor specific biomarkers and design of treatments that target these proteins. The methods may also improve the recovery of tumor specific biomarkers which can inform therapy selection. Such therapies may include small-molecule drugs or monoclonal antibodies. The methods may also improve biomarker testing in individuals suffering from disease and help determine if the individual is a candidate for a certain drug or combination of drugs based on the presence or absence of the biomarker. Additionally, the methods can improve identification of mutations that contribute to the development of resistance to targeted therapy. Consequently, the analysis techniques may reduce unnecessary or untimely therapeutic interventions, patient suffering, and patient mortality.

Therapies can function by helping the immune system destroy cancer cells. For example, certain targeted therapies may mark cancer cells for the immune system to destroy them. Other targeted therapies may support the immune system to work more effectively against cancer. Yet other therapies may stop cancer cells from growing, for example, by interfering with cancer cell surface markers preventing them from dividing. Additionally, therapies can inhibit signals that promote angiogenesis. Such angiogenesis inhibitors prevent blood supply into the tumor thereby, preventing tumor growth. Other targeted therapies can deliver toxic substances to the tumor. Examples include monoclonal antibodies combined with toxins, chemotherapy, or radiation. Some targeted therapies induce apoptosis or deplete cancer of hormones.

In some embodiments, the therapies are PARP inhibitors such as Olaparib (Lynparza), Rucaparib (Rubraca), Niraparib (Zejula), and Talazoparib (Talzenna). In some embodiments the treatment comprises immunotherapies and/or immune checkpoint inhibitors (ICIS) such as anti-pd-1/pd-l1 therapies including pembrolizumab (Keytruda), nivolumab (Opdivo), and cemiplimab (Libtayo), atezolizumab (Tecentriq), durvalumab (Imfinzi), and avelumab (Bavencio). In some embodiments the therapies target mutated forms of the EGFR protein. Such therapies can include osimertinib (Tagrisso), erlotinib (Tarceva), and gefinitib (Iressa).

In some embodiments, therapies can include one or more of the following targeted therapies: abemaciclib (Verzenio), abiraterone acetate (Zytiga), acalabrutinib (Calquence), adagrasib (Krazati), ado-trastuzumab emtansine (Kadcyla), afatinib dimaleate (Gilotrif), alectinib (Alecensa), alemtuzumab (Campath), alitretinoin (Panretin), alpelisib (Piqray), amivantamab-vmjw (Rybrevant), anastrozole (Arimidex), apalutamide (Erleada), asciminib hydrochloride (Scemblix), atezolizumab (Tecentriq), atezolizumab (Tecentriq), avapritinib (Ayvakit), avelumab (Bavencio), axicabtagene ciloleucel (Yescarta), axitinib (Inlyta), belinostat (Beleodaq), belzutifan (Welireg), bevacizumab (Avastin), bexarotene (Targretin), binimetinib (Mektovi), blinatumomab (Blincyto), bortezomib (Velcade), bosutinib (Bosulif), brentuximab vedotin (Adcetris), brexucabtagene autoleucel (Tecartus), brigatinib (Alunbrig), cabazitaxel (Jevtana), cabozantinib-s-malate (Cabometyx), cabozantinib-s-malate (Cometriq), capmatinib hydrochloride (Tabrecta), carfilzomib (Kyprolis), cemiplimab-rwlc (Libtayo), ceritinib (Zykadia), cetuximab (Erbitux), ciltacabtagene autoleucel (Carvykti), cobimetinib fumarate (Cotellic), copanlisib hydrochloride (Aliqopa), crizotinib (Xalkori), dabrafenib (Tafinlar), dabrafenib mesylate (Tafinlar), dacomitinib (Vizimpro), daratumumab (Darzalex), daratumumab and hyaluronidase-fihj (Darzalex Faspro), darolutamide (Nubeqa), dasatinib (Sprycel), denileukin diftitox (Ontak), denosumab (Xgeva), dinutuximab (Unituxin), dostarlimab-gxly (Jemperli), durvalumab (Imfinzi), duvelisib (Copiktra), elacestrant dihydrochloride (Orserdu), elotuzumab (Empliciti), enasidenib mesylate (Idhifa), encorafenib (Braftovi), enfortumab vedotin-ejfv (Padcev), entrectinib (Rozlytrek), enzalutamide (Xtandi), erdafitinib (Balversa), erlotinib hydrochloride (Tarceva), everolimus (Afinitor), exemestane (Aromasin), fam-trastuzumab deruxtecan-nxki (Enhertu), fam-trastuzumab deruxtecan-nxki (Enhertu), fedratinib hydrochloride (Inrebic), fulvestrant (Faslodex), futibatinib (Lytgobi), gefitinib (Iressa), gemtuzumab ozogamicin (Mylotarg), gilteritinib fumarate (Xospata), glasdegib maleate (Daurismo), ibritumomab tiuxetan (Zevalin), ibrutinib (Imbruvica), idecabtagene vicleucel (Abecma), idelalisib (Zydelig), imatinib mesylate (Gleevec), infigratinib phosphate (Truseltiq), inotuzumab ozogamicin (Besponsa), iobenguane I 131 (Azedra), ipilimumab (Yervoy), isatuximab-irfc (Sarclisa), ivosidenib (Tibsovo), ixazomib citrate (Ninlaro), lanreotide acetate (SomatulineDepot), lapatinib ditosylate (Tykerb), larotrectinib sulfate (Vitrakvi), lenvatinib mesylate (Lenvima), letrozole (Femara), lisocabtagene maraleucel (Breyanzi), loncastuximab tesirine-lpyl (Zynlonta), lorlatinib (Lorbrena), lutetium Lu 177 vipivotide tetraxetan (Pluvicto), lutetium Lu 177-dotatate (Lutathera), margetuximab-cmkb (Margenza), midostaurin (Rydapt), mirvetuximab soravtansine-gynx (Elahere), mobocertinib succinate (Exkivity), mogamulizumab-kpkc (Poteligeo), mosunetuzumab-axgb (Lunsumio), moxetumomab pasudotox-tdfk(Lumoxiti), naxitamab-gqgk (Danyelza), necitumumab (Portrazza), neratinib maleate (Nerlynx), nilotinib (Tasigna), niraparib tosylate monohydrate (Zejula), nivolumab (Opdivo), nivolumab and relatlimab-rmbw (Opdualag), obinutuzumab (Gazyva), ofatumumab (Arzerra), olaparib (Lynparza), olutasidenib (Rezlidhia), osimertinib mesylate (Tagrisso), pacritinib citrate (Vonjo), palbociclib (Ibrance), panitumumab (Vectibix), pazopanib hydrochloride(Votrient), pembrolizumab (Keytruda), pemigatinib(Pemazyre), pertuzumab (Perjeta), pertuzumab, trastuzumab, and hyaluronidase-zzxf (Phesgo), pexidartinib hydrochloride (Turalio), pirtobrutinib (Jaypirca), polatuzumab vedotin-piiq (Polivy), ponatinib hydrochloride (Iclusig), pralatrexate (Folotyn), pralsetinib (Gavreto), radium 223 dichloride (Xofigo), ramucirumab (Cyramza), regorafenib (Stivarga), retifanlimab-dlwr (Zynyz), ribociclib (Kisqali), ripretinib (Qinlock), rituximab (Rituxan), rituximab and hyaluronidase human (Rituxan Hycela), romidepsin (Istodax), rucaparib camsylate(Rubraca), ruxolitinib phosphate (Jakafi), sacituzumab govitecan-hziy (Trodelvy), selinexor (Xpovio), selpercatinib (Retevmo), selumetinib sulfate (Koselugo), siltuximab (Sylvant), sirolimus protein-bound particles (Fyarro), sonidegib (Odomzo), sorafenib tosylate (Nexavar), sotorasib (Lumakras), sunitinib malate (Sutent), tafasitamab-cxix (Monjuvi), tagraxofusp-erzs (Elzonris), talazoparib tosylate (Talzenna), tamoxifen citrate (Soltamox), tazemetostat hydrobromide (Tazverik), tebentafusp-tebn (Kimmtrak), teclistamab-cqyv (Tecvayli), temsirolimus (Torisel), tepotinib hydrochloride (Tepmetko), tisagenlecleucel (Kymriah), tisotumab vedotin-tftv (Tivdak), tivozanib hydrochloride (Fotivda), toremifene (Fareston), trametinib (Mekinist), trametinib dimethyl sulfoxide (Mekinist), trastuzumab (Herceptin), tremelimumab-actl (Imjudo), tretinoin (Vesanoid), tucatinib (Tukysa), vandetanib (Caprelsa), vemurafenib (Zelboraf), venetoclax (Venclexta), vismodegib (Erivedge), vorinostat (Zolinza), zanubrutinib (Brukinsa), ziv-aflibercept (Zaltrap).

In one aspect, the disclosure provides a method of preparing a sequencing library from DNA molecules in a sample, the method comprising: (a) providing a first population of DNA molecules from the sample, the first population of DNA molecules comprising double-stranded DNA and single-stranded DNA; (b) ligating a first set of adapters comprising molecular barcodes configured to attach to a plurality of the double-stranded DNA molecules to generate a second population comprising a plurality of adapter-ligated double-stranded DNA molecules, wherein the adapters are ligated to both or one end of the double-stranded DNA molecules, and a plurality of unligated DNA molecules; (c) subjecting the second population to a treatment that denatures and fragments a plurality of the adapter ligated DNA molecules and the unligated DNA molecules to generate a third population of DNA molecules comprising single-stranded DNA molecules having adapter at both, one, and/or neither ends and fragmented single-stranded DNA molecules, wherein the fragmented single-stranded DNA molecules comprise fragments having one and/or no adapter ligated to an end of the fragment; and (d) ligating a second set of adapters to a subset of molecules in the third population which either have an adapter or no adapter ligated to the fragment, thereby generating tagged DNA molecules comprising at least two of: (i) single-stranded adapter-ligated DNA comprising adapters from the first set of adapters ligated to both ends of the molecule, (ii) single-stranded adapter-ligated DNA comprising one adapter from the first set of adapters ligated to one end of the molecule and one adapter from the second set of adapters ligated to the other end of the molecule, and (iii) single-stranded adapter-ligated DNA comprising adapters from the second set of adapters ligated to both ends of the molecule, thereby providing a sequencing library from the population of DNA molecules in the sample. In various embodiments, the adapters are hairpins.

In some embodiments, the first population of DNA molecules comprises double-stranded and single-stranded cell-free DNA (cfDNA).

In some embodiments, the first set of adapters are Y-shaped adapters. In some embodiments, the first set of adapters are protected from the treatment in (c). In some embodiments, the first set of adapters further comprise single-stranded ends that are protected from ligation using modifications comprising 5'OH and/or 3'P. In some embodiments, the first set of adapters comprise single-stranded ends that are protected from ligation not using modifications comprising 5'OH and/or 3'P when T4 PNK is used in (d). In some embodiments, the first set of adapters further comprise single-stranded ends that are protected from ligation using modifications comprising 5' C3 spacer, 5' inverted dideoxy-base, other 5' spacers, 3' C3 spacer, 3' inverted-dT, 3'dideoxy-base, other 3'spacers, when T4 PNK is used in(d). In some embodiments the first set of adapters comprise universal amplification sequences. In some embodiments the molecular barcode differentiates molecules ligated in (b) from molecules ligated in (d).

In some embodiments, T4 PNK is used to phosphorylate the population of DNA molecules prior to ligation in (b). In some embodiments, T4 PNK is used to phosphorylate the population of DNA molecules prior to ligation in (d).

In some embodiments, the treatment that denatures and fragments the second population of DNA molecules comprises at least one of: bisulfite conversion, Tet-assisted bisulfite conversion, Tet-assisted conversion with a substituted borane reducing agent, optionally wherein the substituted borane reducing agent is 2-picoline borane, borane pyridine, tert-butylamine borane, or ammonia borane. In some embodiments, the treatment that denatures and fragments the second population of DNA molecules comprises chemical-assisted conversion with a substituted borane reducing agent, optionally wherein the substituted borane reducing agent is 2-picoline borane, borane pyridine, tert-butylamine borane, or ammonia borane.

In some embodiments, the first set of adapters comprise methylated cytosines to protect them from the treatment. In some embodiments the second set of adapters are `splint' adapters that contain a 5' or 3' overhangs. In some embodiments the second set of adapters comprise universal amplification sequences. In some embodiments the second set of adapters comprise (i) adapters with a double stranded portion and a single stranded overhang comprising a randomer sequence that is 5' of the reverse strand and (ii) adapters with a double stranded portion and a single stranded overhang comprising a randomer sequence that is 3' of the reverse strand. In some embodiments the second set of adapters selectively tag only DNA molecule ends lacking the first adapter sequence.

In some embodiments, the method further comprises amplifying the molecules in (d) (i)-(iii) to generate duplicated DNA molecules. In some embodiments the method further comprises sequencing the amplified DNA molecules to generate sequencing reads. In some embodiments prior to sequencing, the amplified molecules are captured to enrich for one or more target regions. In some embodiments prior to sequencing, the amplified molecules include indexes.

In some embodiments, the sequencing reads are analyzed to resolve unique molecules from PCR duplicates by identifying molecules comprising the same end co-ordinates mapping to a reference sequence and/or molecular barcodes.

In some embodiments, the DNA molecules that have the first adapter at one end and the second adapter at the other end will have greater diversity in the end-coordinates than molecules in (d)(i), wherein said diversity in fragment ends improves accuracy to resolve unique molecules from PCR duplicates. In some embodiments, the DNA molecules that have the second adapter at both ends in (d)(iii) will have greater diversity in the end-coordinates than molecules in (d)(i) and (ii), wherein said diversity improves accuracy to resolve unique molecules from PCR duplicates. In some embodiments, the end-coordinate diversity improves accuracy to resolve unique molecules from PCR duplicates. In some embodiments, the end-coordinates and molecular barcodes improves accuracy to resolve unique molecules from PCR duplicates.

In some embodiments, the forward and reverse strand symmetry improves accuracy to detect methylation status. In some embodiments, the forward and reverse strand symmetry improves accuracy to detect somatic mutations. In some embodiments, for DNA molecules that have the first adapter at both ends in (d)(i), a consensus is generated that accounts for forward and reverse strand symmetry to determine methylation status and/or somatic mutations.

In some embodiments, prior to (b), the DNA molecules in the first population are end-repaired and/or A-tailed.

In another aspect, the disclosure provides a method of analyzing a population of DNA molecules in a sample, the method comprising: (a) ligating a first set of adapters comprising molecular barcodes to at least a subset of DNA molecules within the population of DNA molecules, wherein the adapters are ligated at both ends of the DNA molecules to generate adapter ligated DNA molecules; (b) subjecting the population of DNA molecules to a biochemical treatment that denatures and randomly fragments a plurality of the DNA molecules, thereby producing fragmented DNA molecules that have =< 2 first adapter on their ends; (c) ligating a second set of adapters to the DNA molecules in the population that have < 2 first adapter sequences on their ends to generate a population of tagged DNA molecules comprising at least two of: (i) DNA molecules that have the first adapter at both ends, (ii) DNA molecules that have the first adapter at one end and the second adapter at the other end, (iii) DNA molecules that have the second adapter at both ends; (d) sequencing the population of tagged DNA molecules from (c) to generate sequencing reads; and (e) analyzing the sequencing reads to detect parallel signals associated with disease. In various embodiments, the adapters are hairpins.

In some embodiments, the signals associated with disease further comprise one or more of: short to long cfDNA fragment ratio, differential chromatin architecture, nucleosome structure, epigenetic, and/or genetic information.

In yet another aspect, the disclosure provides a method of preparing a sequencing library from a population of DNA molecules in a sample, the method comprising: (a) ligating a pair of molecular barcodes to a subset of DNA molecules in the population, wherein the pair of molecular barcodes further comprise (i) a first set of molecular barcodes comprising a ligatable end and a 3' single-stranded overhang at the opposite end and (ii) a second set of molecular barcodes comprising a ligatable end and a 5' single-stranded overhang at the opposite end, to generate a plurality of tagged DNA molecules having a molecular barcode at each end; and (b) ligating a set of adapters to a plurality of the tagged DNA molecules and a plurality of non-tagged DNA molecules that did not undergo ligation in (a), to generate (i) adapter-tagged molecules comprising molecular barcodes and (ii) adapter tagged molecules that do not contain the barcode, thereby providing the sequencing library from the population of DNA molecules in the sample.

In some embodiments, the 5' and 3' overhang sequences prevent the pair of barcodes from ligating to each other or self-ligate. In some embodiments the ligatible end comprises a T tail or an A tail. In some embodiments, the ligatible end comprises a blunt end.

In some embodiments, the barcode further comprises an adapter sequence. In some embodiments the adapters are attached to the 3' and 5' single-stranded overhangs of the tagged DNA molecules.

In some embodiments, the set of adapters comprise (i) adapters with a double stranded portion and a single stranded overhang comprising a randomer sequence that is 5' of the reverse strand and (ii) adapters with a double stranded portion and a single stranded overhang comprising a randomer sequence that is 3' of the reverse strand. In some embodiments the adapters are `splint' adapters that contain a 5' or 3' overhangs. In some embodiments, the adapters comprise universal amplification sequences. In some embodiments the method further comprises, amplifying the sequencing library to generate amplified (i) adapter-tagged molecules comprising molecular barcodes and (ii) adapter tagged molecules that do not contain the barcode.

In some embodiments, the method further comprises selectively enriching the library to isolate a subset of the molecules in b(i)(ii). In some embodiments the method further comprises sequencing the library of molecules in b(i)(ii) to generate sequencing reads. In some embodiments the selective enrichment is performed by hybridization or amplification techniques. In some embodiments prior to sequencing, the amplified molecules include indexes.

In some embodiments, the enriched subset of the molecules in b(i)(ii) are associated with a disease. In some embodiments, the disease is cancer, Alzheimer's, hypertriglyceridemia, coronary artery disease.

In yet another aspect, the disclosure provides a method of preparing a sequencing library from a population of DNA molecules in a sample, the method comprising: (a) ligating a first set of adapters comprising a capture-label to at least a subset of DNA molecules to generate a first subset of adapter-ligated DNA molecules comprising a capture-label, wherein the adapters are ligated at both ends of the DNA molecules; (b) separating the adapter ligated DNA molecules comprising the capture-label by contacting the population of DNA molecules with a capture molecule to generate, (i) a first subset of adapter-ligated DNA molecules comprising a capture-label, wherein the label is bound to the capture molecule, (ii) non-adapted DNA molecules; and (c) ligating a second set of adapters to at least a subset of the non-adapted DNA molecules to generate a second subset of adapter-ligated DNA molecules, wherein the adapters are ligated at both ends of the DNA molecules, thereby providing a sequencing library from the populating from DNA molecules in the sample.

In some embodiments, the first set of adapters further comprise y-shape adapters. In some embodiments the first set of adapters further comprise a molecular barcode.

In some embodiments, the capture-label comprises an affinity ligand. In some embodiments the affinity ligand is biotin or photocleavable biotin. In some embodiments, the photocleavable biotin is biotin-UTP. In some embodiments the separating in (b) further comprises affinity purification using at least one capture molecule. In some embodiments, the capture molecule is streptavidin or magnetic beads coated with streptavidin.

In some embodiments, the first set of adapter ligated molecules are subjected to a treatment that digests unmethylated DNA. In some embodiments, the capture molecule are treated with SMRE prior to, during or after streptavidin treatment or application of magnetic beads coated with streptavidin.

In some embodiments, the second set of adapters are `splint' adapters that contain a 5' or 3' overhangs. In some embodiments, the second set of adapters comprise universal amplification sequences. In some embodiments, the second set of adapters comprise (i) adapters with a double stranded portion and a single stranded overhang comprising a randomer sequence that is 5' of the reverse strand and (ii) adapters with a double stranded portion and a single stranded overhang comprising a randomer sequence that is 3' of the reverse strand.

In some embodiments the method further comprises sequencing the library to generate sequencing reads. In some embodiments the method further comprises amplifying the library prior to sequencing. In some embodiments the method further comprises selectively enriching the library prior to sequencing.

In some embodiments, the method further comprises analyzing the sequencing reads to determine the methylation status at one or more genetic loci. In some embodiments the method further comprises analyzing the sequencing reads to determine the molecular topology (e.g., double-stranded originating molecules and single-stranded originating molecules) of the second subset of adapter-ligated DNA molecules in(c).

In some embodiments, the method further comprises analyzing the sequencing reads to detect parallel signals associated with disease. In some embodiments the disease comprises cancer, Alzheimer's, hypertriglyceridemia, or coronary artery disease.

Additional aspects and advantages of the present disclosure will become readily apparent to those skilled in this art from the following detailed description, wherein only illustrative embodiments of the present disclosure are shown and described. As will be realized, the present disclosure is capable of other and different embodiments, and its several details are capable of modifications in various obvious respects, all without departing from the disclosure. Accordingly, the drawings and description are to be regarded as illustrative in nature, and not as restrictive.

### BRIEF DESCRIPTION OF THE FIGURES

The novel features of the disclosure are set forth with particularity in the appended claims. A better understanding of the features and advantages of the present disclosure will be obtained by reference to the following detailed description that sets forth illustrative embodiments, in which the principles of the disclosure are utilized, and the accompanying drawings (also "Figure" and "FIG." herein), of which:

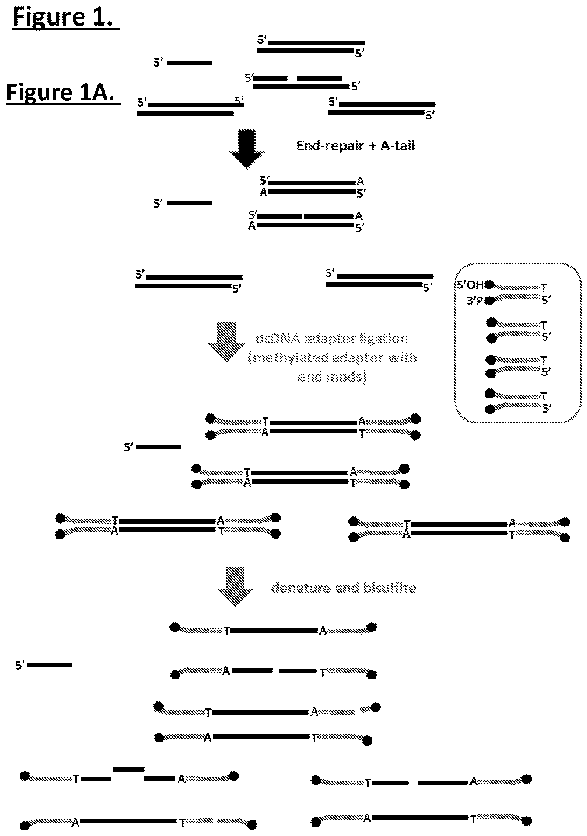

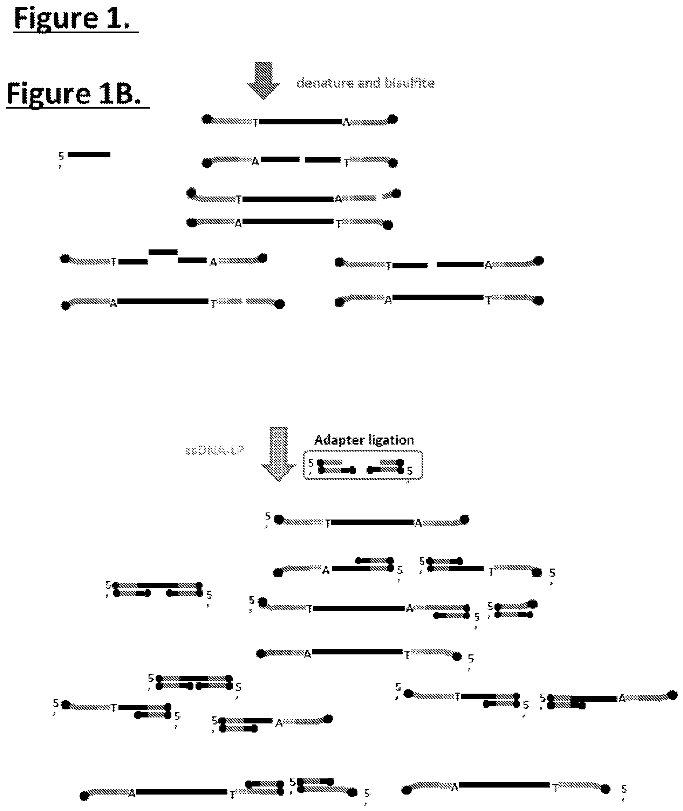

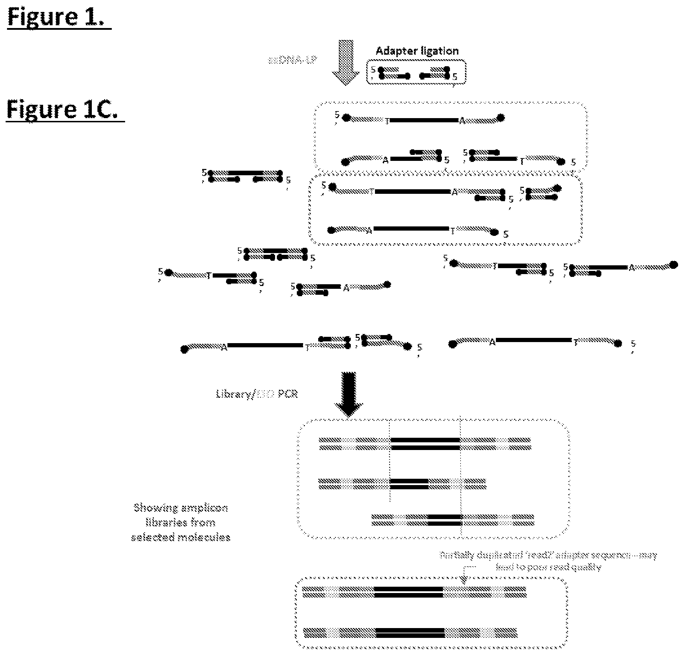

Figure 1. Hybrid dsDNA- and ssDNA-LP for increasing bisulfite-seq recovery. As shown in Figure 1A, shown is a hybrid library preparation workflow to increase bisulfite-seq molecule recovery. In Figure 1B, after denaturation and bisulfite treatment, ssDNA-LP is added. In Figure 1C, PCR amplification and depiction of amplicon libraries from selected molecules.

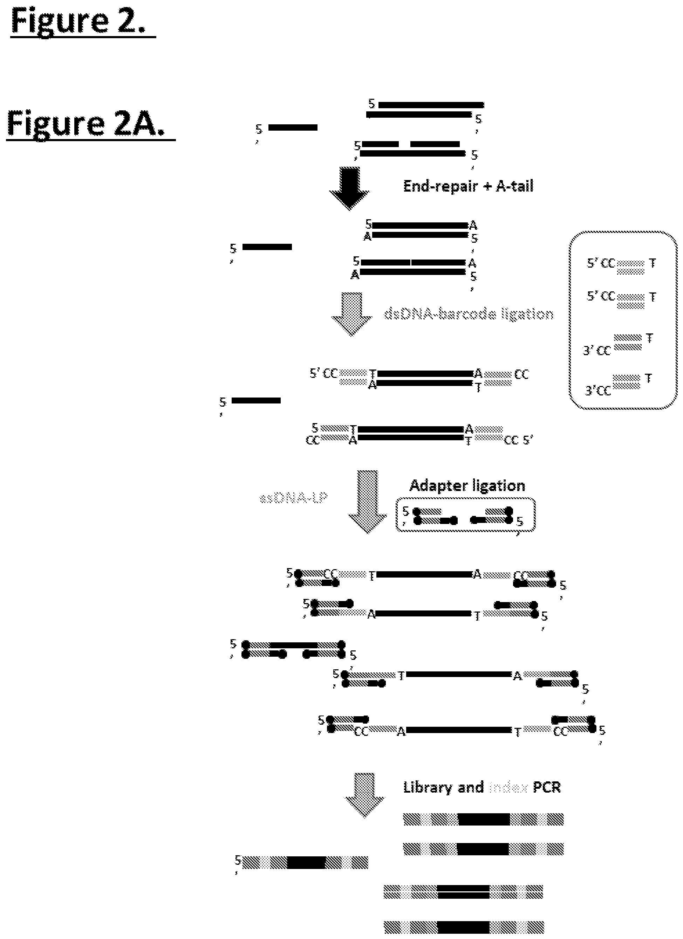

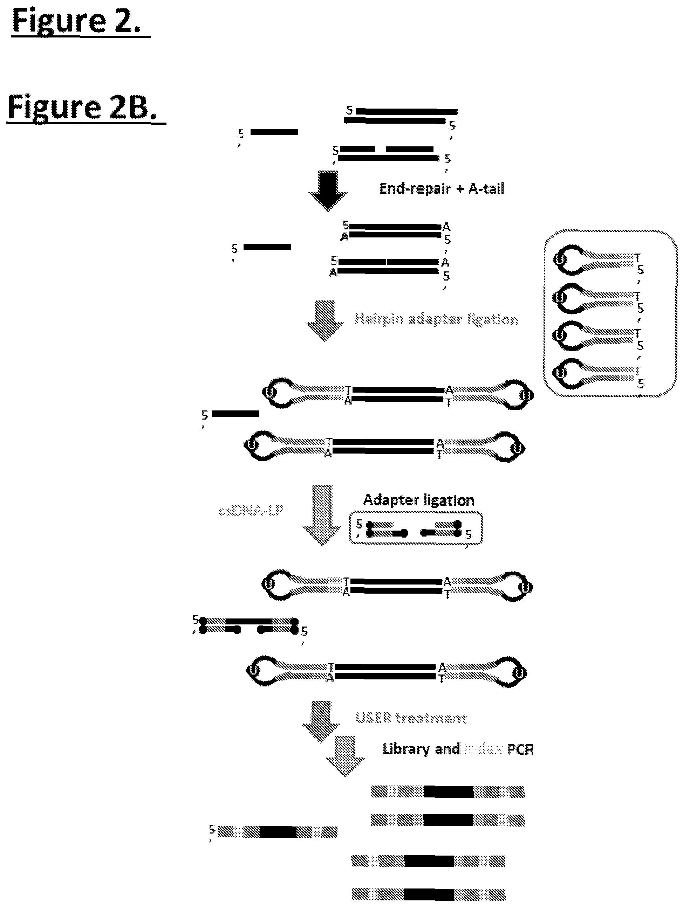

Figure 2. Molecular topology. Shown is a hybrid library preparation workflow to improve single stranded DNA recovery and gain molecule topology information. Figure 2A shows approaches for adding cell free-single stranded DNA (cf-ssDNA) to liquid biopsy workflows using double stranded DNA barcode ligation. Figure. 2B shows approaches for adding cf-ssDNA to liquid biopsy workflows using hairpin dsDNA-ligation.

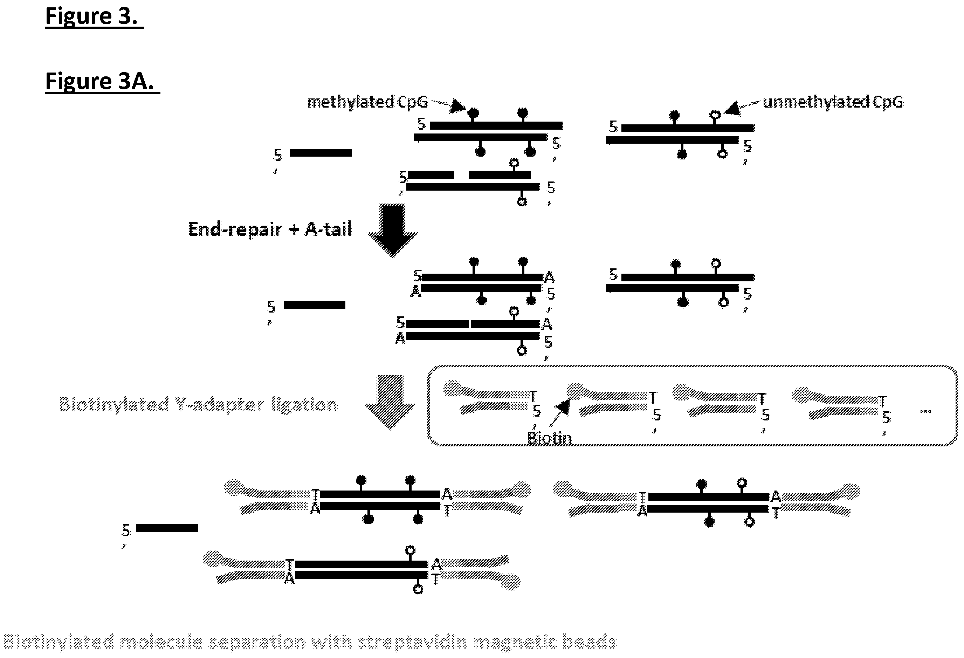

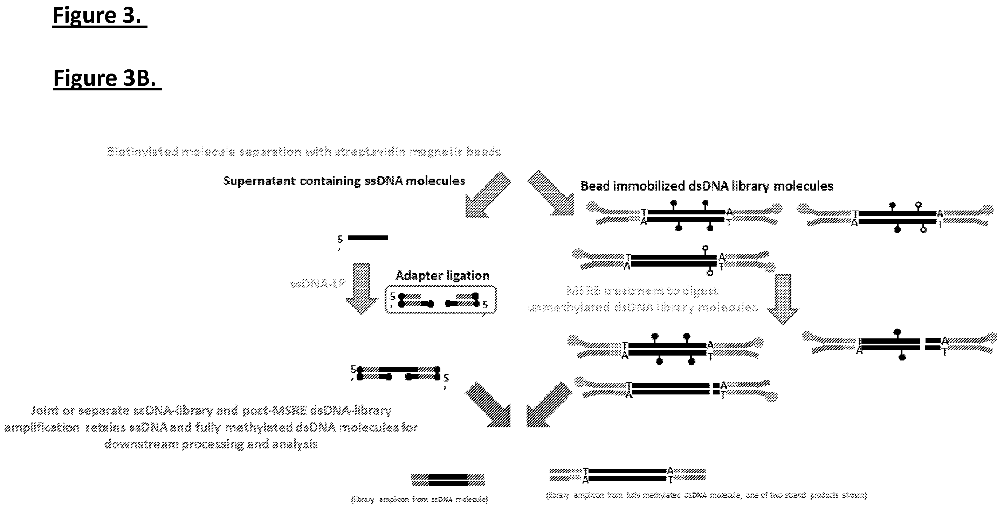

Figure. 3 Split dsDNA MSRE and ssDNA LP processing through biotinylated NGS adapters. As shown in Figure 3A, depicted is a hybrid library preparation workflow to enable multiomic workflows requiring/favoring separate dsDNA-LP and ssDNA-LP treatment. As shown in Figure 3B, the addition of biotinylated NGS adapters is depicted.

### INCORPORATION BY REFERENCE

All publications, patents, and patent applications mentioned in this specification are herein incorporated by reference to the same extent as if each individual publication, patent, or patent application was specifically and individually indicated to be incorporated by reference.

### DETAILED DESCRIPTION

Bisulfite sequencing (Bisulfite-Seq) assays such as Methyl-Seq (Urich, M., Nery, J., Lister, R. et al. MethylC-seq library preparation for base-resolution whole-genome bisulfite sequencing. Nat Protoc 10, 475-483 (2015), incorporated by reference herein) perform dsDNA methylated adapter ligation prior to bisulfite conversion process. With this, the achievable adapter ligation efficiency is very high (60-70%), but a break in the ligated-DNA molecules during bisulfite renders the molecule unamplifiable and unsequencable. Molecular recovery by these methods ranges from <1% -~6% in literature. Newer Bisulfite-Seq methods exist that employ post-bisulfite treatment conversion adaptor tagging to enable sequencing of many of the molecules broken during bisulfite treatment. IDT/Swift's 1D Accel Methyl-NGS and Claret Bio's SRSLY are examples of commercially available ssDNA-LP.

Standard dsDNA-LP has conversion (ligation) efficiency/recovery (>60%). For methylation detection, traditional bisulfite needs to be performed post dsDNA-LP/ligation and results in very low recovery( < 5%). Whereas, bisulfite nicks/breaks DNA molecules (rendering library molecule unamplifiable), but process does not lose DNA (BS-input quant ~ BS-output quant. While ssDNA-LP can be applied post-BS, recovery is limited (15-20%)

When comparing possible outcomes from BS treatment and LP efficiency, for molecules nicked: 0 times, dsDNA-LP will have better recover; 1 time, ssDNA-LP will have better (non-zero) recovery; >2 times, ssDNA-LP will have better (non-zero) recovery

A 'combined' library prep (dsDNA-ligation and backup ssDNA-LP step) could lead to highest overall recovery, for the BS molecule outcomes include: 0 nicks in BS dsDNA-LP preps molecule; 1 nick in BS dsDNA-LP adds one adapter with high efficiency, ssDNA-LP adds 2nd adapter with lower efficiency. Higher than ssDNA-LP alone; 2+ nicks in BS □ ssDNA-LP preps 'inner' fragments (only option) and 'outer' fragments treated as above ('1 nick in BS')

Thus, ssDNA-LP recovery > dsDNA-LP, but depending on prevalence of 0 and 1 nick molecules, combined library prep could yield significantl higher recovery and additionally, there would likely be no need for ssDNA-LP UMIs to fully resolve molecules as 0, 1 nick molecules would get non-random UMI(s) from dsDNA-ligation, + start and stop co-ordinates - 'nick' co-ordinate in 1-nick molecules should impart diversity and 2+ nick molecules should have significant diversity in end co-ordinates from nicks -also less molecules to resolve as 0,1 nick molecules can be fully defined first

Figure. 1 shows a hybrid library preparation workflow to increasing bisulfite-seq molecule recovery. These methods leverage the features of each LP type to boost molecular recovery in Bisulfite-Seq. Pre-bisulfite dsDNA (methylated) adapters are ligated with high efficiency to dsDNA molecules. Bisulfite is then performed, which will break the dsDNA adapted molecules at 0, 1 or > 1 location. Post-bisulfite, a ssDNA-LP is performed to selectively rescue (adaptor tag) the molecule fragments that have <2 dsDNA adapter sequences on its ends. If a dsDNA-adapter tagged molecule was not broken in bisulfite then it will be unaffected by the ssDNA LP, while if the molecule contained 1 break, the ssDNA prep will adapter tag the broken end, preparing the molecule for sequencing - the resultant library molecule will have a dsDNA-LP arising adapter at one end and an ssDNA-LP arising adapter at the other end. If the DNA molecules are broken in more than one place by bisulfite, then molecules with bisulfite-generated breaks at both ends will have ssDNA-LP arising adapters on both ends. Non-random molecular barcodes may be used with the dsDNA-LP adapters, which enables molecule resolution power for 0 break and 1 break DNA fragments in sequencing. Additionally, with this the adapter type added to each end of the DNA molecule, it can be determined whether the molecule originated from fragment end or bisulfite break. This info is identifiable in sequencing (looking at presence/absence of molecular barcodes).

An aspect of the present disclosure provides methods of preparing a sequencing library from DNA molecules in a sample using a hybrid library prep method. Prior to bisulfite treatment, tag double-stranded, end-prepared DNA molecules with Y-shaped NGS adapters with molecular barcodes through ligation (T4 DNA ligase). Cytosine bases in the Y-shaped adapters are protected from bisulfite conversion and single-stranded ends are protected from ligation. Perform bisulfite conversion, which will fragment a portion of the NGS-adapted DNA molecules. Subject the post-bisulfite converted DNA to a ssDNA-LP using 'splint' adapters that contain a 5' or 3' overhang and the appropriate NGS universal amplification sequences, that are also contained in the Y-adapters. The splint adapters will selectively tag only DNA ends lacking Y-adapter sequence. This can occur because first ligation failed, or bisulfite fragmented the DNA molecule - the latter is the significant population that this invention aims to rescue. After splint-adapter ssDNA ligation, all the molecules with adapters (either type) on 5' and 3' end are amplified with universal primers and processed downstream for NGS - hybrid capture enrichment may be performed before sequencing. Post-sequencing, the analysis identifies and resolves molecules through DNA end co-ordinates and molecular barcodes if present on the molecule/read. Bisulfite-fragmentation will generate more diversity in fragment ends and thus, for bisulfite-fragmented molecules (no molecular barcode present) molecular resolution is feasible without molecular barcodes. Completely non-fragmented molecules will have the least amount of diversity in the end-coordinates, and they will have two molecular barcode tags present to that are used in conjunction with fragment ends to resolve molecules. Molecules in which one end is fragmented while have contain a molecular barcode on the low diversity end - molecules can be identified by combination of fragment ends and single molecular barcode. In this way, the 'hybrid' library prep protocol also improves the accuracy of molecular resolution in bisulfite sequencing workflows.

Modifications to the ssDNA-ends of the Y-adapters.

In commercial ssDNA-LP 'splint adapter ligation' kits, there is a step cocurrent with ligation that phosphorylates 5' ends and optionally removes 3' phosphate groups, preparing them for ligation. This step can be skipped for the hybrid workflow described herein, as it is performed during the upstream 'end-repair' that prepares dsDNA for Y-adapter ligation. In this case 5' hydroxyl group and 3' phosphate group are simplest and most economical modifications to employ. However, if the ssDNA-LP T4 PNK step is left in the protocol, these two modifications cannot be used as they are substrates for T4 PNK activity, which will convert them to ligatable ends. In protocols with a ssDNA-LP PNK step, such suitable modifications to the ssDNA-ends of the Y-adapters are 5' C3 spacer, 5' inverted dideoxy-base, other 5' spacers, 3' C3 spacer, 3' inverted-dT, 3'dideoxy-base, other 3'spacers, as these will all prevent/inhibit T4 PNK activity.

### Loss of ssDNA molecules and DNA molecule topology information (ssDNA- or dsDNA-originating) in genetic sequencing workflows.

Cell free DNA (cfDNA) predominantly exists as dsDNA, which are sequenced effectively by dsDNA-LP methods. However, the minor and potentially biologically significant cf-ssDNA, as well as cf-dsDNA molecules that are denatured to ssDNA prior to LP (extraction process is slightly denaturing) are not sequenced by the dsDNA-LP process. Using a ssDNA-LP process alone to recover these ssDNA and dsDNA is not ideal, as the molecular recovery relatively low and information regarding originating molecule topology (ssDNA or dsDNA) is not retained.

Figure 2, including Figs. 2A-B show a hybrid library preparation workflow to improve single stranded DNA recovery and gain molecule topology information. Figure 2A. shows an exemplary method to recover cf-ssDNA from liquid biopsy workflows using double stranded DNA barcode ligation. In this embodiment double stranded DNA molecules are ligated with dsDNA-identifying barcode tag, sequencing reads are then analyzed bioinformatically to extract the original topology(single stranded or double stranded) of the DNA molecule. After ligation of the dsDNA-barcode tags, the sample is then subjected to a ssDNA-LP. Through this hybrid LP method both DNA molecules originating as ssDNA or dsDNA will be amplified and sequenced, and specific dsDNA-barcode tags will identify the original topology of the molecule (ss or ds).

The dsDNA-identifying barcode tag informs that the molecule originated as dsDNA. Specifically, a plurality of dsDNA-barcode tags may be used, and they may also serve as molecular barcodes. The dsDNA ligation may just add 'dsDNA-barcode tags' or it can add the barcode tags as part of Y-adapter. After ligation of the dsDNA-barcode tags, the sample is then subjected to a ssDNA-LP. In the 'dsDNA-barcode' ligation step, if only 'tags' are added the ssDNA-LP will add amplification and sequencing adapters to these molecules, as well as the ssDNA-originating molecules, that will be resolvable in sequencing as they do not contain the dsDNA-barcode tags. If Y-adapter (with dsDNA-barcodes) is ligated to dsDNA molecules in the first step, then the same Y-adapter modifications discussed above to prevent undesired ligation in the ssDNA-LP apply here. Figure 2B shows an alternative method to recover cf-ssDNA from liquid biopsy workflows using hairpin adapter ligation. In this embodiment the dsDNA molecules are ligated with T-tailed (optional) with hairpin NGS adapters (such as that used NEBNext kits) modified to have molecular barcodes and subjected to ssDNA LP method using splint adapter ligation. Example 2 shows an exemplary workflow.

### Incompatibility with certain multiomic workflows with std LP methods.

Methylation-Sensitive Restriction Enzyme (MSREs) sequencing workflows have advantage in methylation analysis in that they yield high molecular recovery. To achieve highest accuracy in methylation calling with lowest sequencing burden a dsDNA-LP is used and the MSRE treatment occurs after Y-adapter ligation, but before amplification. As such, only molecules with fully methylated MSRE recognition sites are amplified and sequenced. Such a workflow requires dsDNA-LP, preventing/hampering concurrent detection of other omic signals that require/favor ssDNA-LP workflows, such as potentially fragmentomics looking at short to long cfDNA fragment ratios, and other fragmentomics analysis looking at subnucleosomal cfDNA molecules, and subnucleosomal disease-defining features in cell free DNA, and nucleosome footprints that may inform tissue-of-origin.

Figure 3 shows a hybrid library preparation workflow to enable multiomic workflows requiring/favoring separate dsDNA-LP and ssDNA-LP treatment. To enable MSRE treatment removal from sequencing libraries of unmethylated (dsDNA) molecules, while preparing ssDNA molecules for sequencing, an enrichable reagent is incorporated into the dsDNA ligation and/or MSRE cleavage repair step to enable separate those substrates from ssDNA molecules that can then be prep for sequencing through a ssDNA-LP step. If this enrichment/separation step is not taken, MSRE cleavage products would be prepared for sequencing through an undesirable ssDNA-LP step.

### Use of biotinylated Y-adapters to separate ssDNA material.

Double stranded DNA molecules, after end-repair/tailing are ligated with biotinylated Y-adapters. Streptavidin-magnetic beads and a magnet are applied to immobilize/pull down the ligated dsDNA and the ssDNA molecules in the supernatant are removed to separate reaction vessel. In parallel reactions, MSRE treatment is applied to the adapter ligated dsDNA, the resultant library, devoid of molecules unmethylated at MSRE sites is amplified for downstream processing. The ssDNA-containing reaction vessel is then subjected to a ssDNA-LP. After the ligation step in the ssDNA-LP step it is safe to combine the ssDNA library with the post-MSRE dsDNA library for co-library amplification and/or hybrid capture and/or sequencing.

Alternatively, in some embodiments, biotin dATP may be added in the A-tailing step to tag the dsDNA library molecules with biotin. This method of biotinylating the dsDNA molecules may have a negative impact on amplification efficiency and resultantly, process sensitivity. Use of photocleavable-biotin or standard biotin and addition of a cleavable base (i.e., uracil) in the Y-adapter is an optional method that would enable release of the dsDNA library molecules from immobilization, after the ssDNA molecules have been removed from the sample. This could be beneficial if immobilization causes steric effects that inhibit MSRE and/or PCR amplification efficiency of the dsDNA library molecules.

### Specific biotinylation of MSRE products to remove them from ssDNA-LP.

In this method the Y-adapter may contain ssDNA-end modifications as previously discussed that prevent their ends from being modified during downstream enzymatic steps The dsDNA molecules are end-repaired/A-tailed and ligated to the modified Y-adapters, after which MSRE treatment is performed. After MSRE treatment, the DNA is again end-repaired and A-tailed, but this time containing a biotinylated nucleotide(s) - use of A-tailing with biotin-dATP is needed to tag MSRE reactions that leave 5' overhang (such as HpaII). The Y-adapter ssDNA ends, as well as the ssDNA molecules in the sample should be inert to this end-repair/A-tailing. Streptavidin-magnetic beads and a magnet are applied to immobilize/pull down the MSRE products and the undigested dsDNA library molecules and ssDNA molecules are removed as supernatant to a new reaction vessel. The ssDNA-LP is carried out on the new reaction vessel, selectively preparing the ssDNA molecules. In the same tube co-amplification of both post-MSRE dsDNA library and ssDNA library molecules is carried out and the product is taken for downstream processing - such as hybrid capture and/or sequencing.

The present disclosure provides methods to develop next generation sequencing libraries useful for cancer screening test (cancer-specific or multi-cancer test) and incorporate into a targeted sequencing workflow (e.g., hybrid capture panel) relevant genomic regions associated with disease. To monitor genetic and epigenetic alterations associated treatment response, resistance and/or identify markers of disease initiation, progression, and metastasis in cfDNA or tissue.

### 1. Samples

A sample can be any biological sample isolated from a subject. Samples can include body tissues, such as known or suspected solid tumors, whole blood, platelets, serum, plasma, stool, red blood cells, white blood cells or leucocytes, endothelial cells, tissue biopsies, cerebrospinal fluid synovial fluid, lymphatic fluid, ascites fluid, interstitial or extracellular fluid, the fluid in spaces between cells, including gingival crevicular fluid, bone marrow, pleural effusions, cerebrospinal fluid, saliva, mucous, sputum, semen, sweat, urine. Samples are preferably body fluids, particularly blood and fractions thereof, and urine. A sample can be in the form originally isolated from a subject or can have been subjected to further processing to remove or add components, such as cells, or enrich for one component relative to another. Thus, a preferred body fluid for analysis is plasma or serum containing cell-free nucleic acids.

The volume of plasma can depend on the desired read depth for sequenced regions. Exemplary volumes are 0.4-40 mL, 5-20 mL, 10-20 mL. For example, the volume can be 0.5 mL, 1 mL, 5 mL 10 mL, 20 mL, 30 mL, or 40 mL. A volume of sampled plasma may be for example 5 to 20 mL.

A sample can comprise various amount of nucleic acid that contains genome equivalents. For example, a sample of about 30 ng DNA can contain about 10,000 haploid human genome equivalents and, in the case of cell-free DNA, about 200 billion individual nucleic acid molecules. Similarly, a sample of about 100 ng of DNA can contain about 30,000 haploid human genome equivalents and, in the case of cell-free DNA, about 600 billion individual molecules. Some samples contain 1-500, 2-100, 5-150 ng cell-free DNA, e.g., 5-30 ng, or 10-150 ng cell-free DNA.

A sample can comprise nucleic acids from different sources. For example, a sample can comprise germline DNA or somatic DNA. A sample can comprise nucleic acids carrying mutations. For example, a sample can comprise DNA carrying germline mutations and/or somatic mutations. A sample can also comprise DNA carrying cancer-associated mutations (e.g., cancer-associated somatic mutations).

Exemplary amounts of cell-free nucleic acids in a sample before amplification range from about 1 fg to about 1 ug, e.g., 1 pg to 200 ng, 1 ng to 100 ng, 10 ng to 1000 ng. For example, the amount can be up to about 600 ng, up to about 500 ng, up to about 400 ng, up to about 300 ng, up to about 200 ng, up to about 100 ng, up to about 50 ng, or up to about 20 ng of cell-free nucleic acid molecules. The amount can be at least 1 fg, at least 10 fg, at least 100 fg, at least 1 pg, at least 10 pg, at least 100 pg, at least 1 ng, at least 10 ng, at least 100 ng, at least 150 ng, or at least 200 ng of cell-free nucleic acid molecules. The amount can be up to 1 femtogram (fg), 10 fg, 100 fg, 1 picogram (pg), 10 pg, 100 pg, 1 ng, 10 ng, 100 ng, 150 ng, or 200 ng of cell-free nucleic acid molecules. The method can comprise obtaining 1 femtogram (fg) to 200 ng.

An exemplary sample is 5-10 ml of whole blood, plasma or serum, which includes about 30 ng of DNA or about 10,000 haploid genome equivalents.

Cell-free nucleic acids are nucleic acids not contained within or otherwise bound to a cell or in other words nucleic acids remaining in a sample of removing intact cells. Cell-free nucleic acids include DNA, RNA, and hybrids thereof, including genomic DNA, mitochondrial DNA, siRNA, miRNA, circulating RNA (cRNA), tRNA, rRNA, small nucleolar RNA (snoRNA), Piwi-interacting RNA (piRNA), long non-coding RNA (long ncRNA), or fragments of any of these. Cell-free nucleic acids can be double-stranded, single-stranded, or a hybrid thereof. Double-stranded DNA molecules at least some of which have single-stranded overhangs are a preferred form of cell-free DNA for any method disclosed herein. A cell-free nucleic acid can be released into bodily fluid through secretion or cell death processes, e.g., cellular necrosis and apoptosis. Some cell-free nucleic acids are released into bodily fluid from cancer cells e.g., circulating tumor DNA, (ctDNA). Others are released from healthy cells.

A cell-free nucleic acid can have one or more epigenetic modifications, for example, a cell-free nucleic acid can be acetylated, methylated, ubiquitinylated, phosphorylated, sumoylated, ribosylated, and/or citrullinated.

Cell-free nucleic acids have a size distribution of about 100-500 nucleotides, particularly 110 to about 230 nucleotides, with a mode of about 168 nucleotides and a second minor peak in a range between 240 to 440 nucleotides.

Cell-free nucleic acids can be isolated from bodily fluids through a partitioning step in which cell-free nucleic acids, as found in solution, are separated from intact cells and other non-soluble components of the bodily fluid. Partitioning may include techniques such as centrifugation or filtration. Alternatively, cells in bodily fluids can be lysed and cell-free and cellular nucleic acids processed together. Generally, after addition of buffers and wash steps, nucleic acids can be precipitated with an alcohol. Further clean up steps may be used such as silica based columns to remove contaminants or salts. Non-specific bulk carrier nucleic acids, for example, may be added throughout the reaction to optimize certain aspects of the procedure such as yield.

After such processing, samples can include various forms of nucleic acid including double-stranded DNA, single-stranded DNA and single-stranded RNA. Optionally, single stranded DNA and RNA can be converted to double stranded forms so they are included in subsequent processing and analysis steps.

### 2. Linking sample nucleic acid molecules to adapters

Nucleic acids present in a sample with or without prior processing as described above typically contain a substantial portion of molecules in the form of partially double-stranded molecules with single-stranded overhangs. Such molecules can be converted to blunt-ended double-stranded molecules by treating with one or more enzymes to provide a 5'-3' polymerase and a 3'-5' exonuclease (or proof-reading function), in the presence of all four standard nucleotide types as shown in Fig. 1, upper. Such a combination of activities can extend strands with a recessed 3' end so they end flush with the 5' end of the opposing strand (in other words generating a blunt end) or can digest strands with 3' overhangs so they are likewise flush with the 5' end of the opposing strand. Both activities can optionally be conferred by a single polymerase. The polymerase is preferably heat-sensitive so that its activity can be terminated when the temperature is raised. Klenow large fragment and T4 polymerase are examples of suitable polymerase.

The one or more enzymes conferring 5'-3' polymerase and a 3'-5' exonuclease activity are preferably denatured by raising the temperature or otherwise. For example, denaturation can be affected by raising the temperature to e.g., to 75°-80° C. The samples are then acted on by a polymerase lacking a proof-reading function (Fig. 1 middle). This polymerase is preferably thermostabile such as to remain active at the elevated temperature. Taq, Bst large fragment and Tth polymerases are examples of such a polymerase. The second polymerase effects a non-templated addition of a single nucleotide to the 3' ends of blunt-ended nucleic acids. Although the reaction mixture typically contains equal molar amounts of each of the four standard nucleotide types from the prior step, the four nucleotide types are not added to the 3' ends in equal proportions. Rather A is added most frequently, followed by G followed by C and T.

In certain embodiments, after tailing of the sample molecules, and with or without subsequent purification of the tailed sample molecules, the tailed sample molecules are contacted with adapters tailed with complementary T and C nucleotides at one end of the adapters (Fig. 1, lower). Adapters are typically formed by separate synthesis and annealing of their respective strands. The additional T and C tails can thus be added as an extra nucleotide in synthesis of one of the strands. Typically adapters tailed with G and A are not included because although these adapters might anneal with sample molecules tailed with C and T respectively, they would also anneal with other adapters. Adapter molecules and sample molecules bearing complementary nucleotides (i.e., T-A and C-G) at their 3' ends anneal and can be ligated to one another. The percentage of C-trailed adapters relative to T-tailed adapters ranges from about 5-40% by moles, for example, 10-35%, 15-25%, 20-35%, 25-35% or about 30%. Because the non-template directed addition of a single nucleotide to the 3' ends of sample molecules does not proceed to completion, the sample also contains some blunt-ended sample molecules without tailing. These molecules can be recovered by also supplying the sample with adapters having one and preferably only one blunt end. Blunt end adapters are usually supplied at a molar ratio of 0.2-20%, or 0.5-15% or 1-10% of adapters with T- and C-tailed adapters. Blunt-ended adapters can be provided at the same time, before or after the T- and C-tailed adapters. Blunt-ended adapters ligated with blunt-ended sample molecules again resulting in sample molecules flanked on both sides by adapters. These molecules lack the AT or C-G nucleotide pairs between sample and adapters present when tailed sample molecules are ligated to tailed adapters.

The adapters used in these reactions may have one and only one end tailed with T or C or one and only one end blunt so that they can ligate with sample molecules in only one orientation. The adapters can be for example Y-shaped adapters in which one end is tailed or blunt and the other end has two single strands. Exemplary Y-shaped adapters have sequences as follows with (6 bases) indicating a tag. The upper oligonucleotide includes a single base T tail.

Customized combinations of such oligonucleotide including oligonucleotides with both T and C tails can be synthesized for use in the present methods.

A truncated version of these adapter sequences has been described by Rohland et al., Genome Res. 2012 May; 22(5): 939-946.

Adapters can also be bell-shaped with only one end, which is tailed or blunt. Adapters can include a primer binding site for amplification, a binding site for a sequencing primer, and/or a nucleic acid tag for purposes of identification. The same or different adapters can be used in in a single reaction.

When adapters include an identification tag and nucleic acids in a sample are attached to adapters at each end, the number of potential combinations of identifiers increases exponentially with the number of unique tags supplied (i.e., nn combinations, where n is the number of unique identification tags). In some methods, the number of combinations of unique tags is sufficient that it is statistically probable that all or substantially all (e.g., at least 90%) of different double-stranded DNA molecules in the sample receive a different combination of tags. In some methods, the number of unique combinations of identifier tags is less than the number of unique double-stranded DNA molecules in the sample (e.g., 5-10,000 different tag combinations).

A kit providing suitable enzymes for performing the above methods is the NEBNext^{®} Ultra^{™} II DNA Library Prep Kit for Illumina^{®}. The kit provides the following reagents.

NEBNext Ultra II End Prep Enzyme Mix, NEBNext Ultra II End Prep Reaction Buffer, NEBNext Ligation Enhancer, NEBNext Ultra II Ligation Master Mix -20, NEBNext^{®} Ultra II Q5^{®} Master Mix.

The blunt-ending and tailing of sample nucleic acids can be performed in a single-tube. Blunt-ended nucleic acids need not be separated from the enzyme(s) performing the blunt ending before the tailing reaction occurs. Optionally, all enzymes, nucleotides and other reagents are supplied together before the blunt ending reaction occurs. Supplying together means that all are introduced in the sample sufficiently proximate in time such that all are present when the sample incubation occurs for blunt ending to take place. Optionally, nothing is removed from the samples after supplying the enzymes, nucleotides and other reagents at least until both the blunt ending and end tailing incubations have been completed. Often, the end tailing reaction is performed at a higher temperature than the blunt ending reaction. For example, the blunt ending reaction can be performed at ambient temperature in which the 5'-3' polymerase and 3'-5' exonuclease are active and the thermostabile polymerase is inactive or minimally active, and the end tailing reaction performed at an elevated temperature, such as over 60°C, when the 5'-3' polymerase and 3'-5' exonuclease are inactive and the thermostabile polymerase is active.

### 3. Amplification

Sample nucleic acids flanked by adapters can be amplified by PCR and other amplification methods typically primed from primers binding to primer binding sites in adapters flanking a nucleic acid to be amplified. Amplification methods can involve cycles of extension, denaturation and annealing resulting from thermocycling or can be isothermal as in transcription mediated amplification. Other amplification methods include the ligase chain reaction, strand displacement amplification, nucleic acid sequence-based amplification, and self-sustained sequence-based replication.

Preferably, the present methods result in at least 75, 80, 85, 90 or 95% of double-stranded nucleic acids in the sample being linked to adapters. Preferably use of T- and C-tailing increases the percentage of double-stranded nucleic acids in the sample linked to adaptors relative to control methods performed with T-tailed adapters alone by at least 1, 2, 3, 4, 5, 6, 7, 8, 9 or 10% (an increase of yield from 75% to 80% being considered a 5% increase). Preferably, use of T- and C-tailing in combination with blunt-ended adaptors increase the percentage of double-stranded nucleic acids linked to adaptors by at least 5, 10, 15, 20 or 25%. The percentage of nucleic acids linked to adaptors can be determined by comparative gel electrophoresis of the original sample and the processed sample after linkage to adapters has been completed.

Preferably, the present methods result in at least 75, 80, 85, 90 or 95% of available double-stranded molecules in the sample being sequenced. Preferably the use of T- and C-tailing increases the percentage of double-stranded nucleic acids in the sample being sequenced relative to control methods performed with T-tailed adapters alone by at least 1, 2, 3, 4, 5, 6, 7, 8, 9 or 10%. Preferably the use of T- and C-tailing in combination with blunt ended adaptors increases the percentage of double-stranded nucleic acid in the sample being sequenced relative to control methods performed with T-tailed adaptors along by at least 5, 10, 15, 20 or 25%. The percentage of nucleic acids being sequenced can be determined by comparing the number of molecules actually sequenced based on the number that could have been sequenced based on the input nucleic acids and regions of the genome targeted for sequencing.

### 4. Tags

Tags providing molecular identifiers or bar codes can be incorporated into or otherwise joined to adapters by ligation, overlap extension PCR among other methods. Generally, assignment of unique or non-unique identifiers, or molecular barcodes in reactions follows methods and systems described by US patent applications 20010053519, 20030152490, 20110160078, and U.S. Pat. No. 6,582,908 and U.S. Pat. No. 7,537,898.

Tags can be linked to sample nucleic acids randomly or non-randomly. In some cases, they are introduced at an expected ratio of unique identifiers to microwells. For example, the unique identifiers may be loaded so that more than about 1, 2, 3, 4, 5, 6, 7, 8, 9, 10, 20, 50, 100, 500, 1000, 5000, 10000, 50,000, 100,000, 500,000, 1,000,000, 10,000,000, 50,000,000 or 1,000,000,000 unique identifiers are loaded per genome sample. In some cases, the unique identifiers may be loaded so that less than about 2, 3, 4, 5, 6, 7, 8, 9, 10, 20, 50, 100, 500, 1000, 5000, 10000, 50,000, 100,000, 500,000, 1,000,000, 10,000,000, 50,000,000 or 1,000,000,000 unique identifiers are loaded per genome sample. In some cases, the average number of unique identifiers loaded per sample genome is less than, or greater than, about 1, 2, 3, 4, 5, 6, 7, 8, 9, 10, 20, 50, 100, 500, 1000, 5000, 10000, 50,000, 100,000, 500,000, 1,000,000, 10,000,000, 50,000,000 or 1,000,000,000 unique identifiers per genome sample.

In some cases, unique identifiers may be predetermined or random or semi-random sequence oligonucleotides. In other cases, a plurality of barcodes may be used such that barcodes are not necessarily unique to one another in the plurality. In this example, barcodes may be ligated to individual molecules such that the combination of the bar code and the sequence it may be ligated to creates a unique sequence that may be individually tracked. As described herein, detection of non-unique barcodes in combination with sequence data of beginning (start) and end (stop) portions of sequence reads may allow assignment of a unique identity to a particular molecule. The length, or number of base pairs, of an individual sequence read may also be used to assign a unique identity to such a molecule. As described herein, fragments from a single strand of nucleic acid having been assigned a unique identity may thereby permit subsequent identification of fragments from the parent strand.

Polynucleotides in a sample can be tagged with a sufficient number of different tags so that there is a high probability (e.g., at least 90%, at least 95%, at least 98%, at least 99%, at least 99.9% or at least 99.99%) that all polynucleotides mapping to a particular genomic region bear a different identifying tag (molecules within the region are substantially uniquely tagged). The genomic region to which the polynucleotides map can be, for example, (1) the entire panel of genes being sequenced, (2) some portion of that panel, such as mapping within a single gene, exon or intron, (3) a single nucleotide coordinate (e.g., at least one nucleotide in the polynucleotide maps to the coordinate, for example, the start position, stop position, mid-point or anywhere between) or (4) a particular pair of start/stop (begin/end) nucleotide coordinates. The number of different identifiers (tag counts) necessary to substantially uniquely tag polynucleotides is a function of how many original polynucleotide molecules in the sample that map to the region. This, in turn, is a function of several factors. One factor is the total number of haploid genome equivalents included in the assay. Another factor is the average size of the polynucleotide molecules. Another factor is the distribution of the molecules across the region. This, in turn, can be a function of the cleavage pattern - one may expect cleavage to occur primarily between nucleosomes so that more polynucleotides map across a nucleosome location than between nucleosomes. Another factor is the distribution of barcodes in the pool and the ligation efficiency of individual barcodes, potentially causing differences in affective concentration of one barcode versus another. Another factor is the size of the region within which the molecules to be uniquely tagged are confined (e.g., same start/stop or same exon).

The identifier can be a single barcode attached to one end of a molecule, or two barcodes, each attached to different ends of the molecule. Attaching barcodes independently to both ends of a molecule increases by square the number of possible identifiers. In this case the number of different barcodes is selected such that the combination of barcodes on each end of a particular polynucleotide has a high probability of being unique with respect to other polynucleotides mapping to the same selected genomic region.

In certain embodiments, the number of different identifiers or barcode combinations (tag count) used can be at least any of 64, 100, 400, 900, 1400, 2500, 5625, 10,000, 14,400, 22,500 or 40,000 and no more than any of 90,000, 40,000, 22,500, 14,400 or 10,000. For example, the number of identifiers or barcode combinations can be between 64 and 90,000, between 400 and 22,500, 400 and 14,400 or between 900 and 14,400.

In a sample comprising fragmented genomic DNA, e.g., cell-free DNA (cfDNA), from a plurality of genomes, there is some likelihood that more than one polynucleotide from different genomes will have the same start and stop positions ("duplicates" or "cognates"). The probable number of duplicates beginning at any position is a function of the number of haploid genome equivalents in a sample and the distribution of fragment sizes. For example, cfDNA has a peak of fragments at about 160 nucleotides, and most of the fragments in this peak range from about 140 nucleotides to 180 nucleotides. Accordingly, cfDNA from a genome of about 3 billion bases (e.g., the human genome) may be comprised of almost 20 million (2x107) polynucleotide fragments. A sample of about 30 ng DNA can contain about 10,000 haploid human genome equivalents. (Similarly, a sample of about 100 ng of DNA can contain about 30,000 haploid human genome equivalents.) A sample containing about 10,000 (104) haploid genome equivalents of such DNA can have about 200 billion (2x1011) individual polynucleotide molecules. It has been empirically determined that in a sample of about 10,000 haploid genome equivalents of human DNA, there are about 3 duplicate polynucleotides beginning at any given position. Thus, such a collection can contain a diversity of about 6x1010-8x1010 (about 60 billion-80 billion e.g., about 70 billion (7x1010)) differently sequenced polynucleotide molecules.

The probability of correctly identifying molecules is dependent on initial number of genome equivalents, the length distribution of sequenced molecules, sequence uniformity and number of tags. The number can be calculated using a Poisson distribution. When the tag count is equal to one, that is, equivalent to having no unique tags or not tagging. Table 1 below lists the probability of correctly identifying a molecule as unique assuming a typical cell-free size distribution as above.

**Table 1. Probability of correctly identifying a molecule**

| Tag Count | Tag %Correctly uniquely identified |
|---|---|
| 1000 human haploid genome equivalents | |
| 1 | 96.9643 |
| 4 | 99.2290 |
| 9 | 99.6539 |
| 16 | 99.8064 |
| 25 | 99.8741 |
| 100 | 99.9685 |
| | |

| 3000 human haploid genome equivalents | |
|---|---|
| 1 | 91.7233 |
| 4 | 97.8178 |
| 9 | 99.0198 |
| 16 | 99.4424 |
| 25 | 99.6412 |
| 100 | 99.9107 |

In this case, upon sequencing the genomic DNA, it may not be possible to determine which sequence reads are derived from which parent molecules. This problem can be diminished by tagging parent molecules with a sufficient number of unique identifiers (e.g., the tag count) such that there is a likelihood that two duplicate molecules, i.e., molecules having the same start and stop positions, bear different unique identifiers so that sequence reads are traceable back to particular parent molecules. One approach to this problem is to uniquely tag every, or nearly every, different parent molecule in the sample. However, depending on the number of haploid gene equivalents and distribution of fragment sizes in the sample, this may require billions of different unique identifiers.

This method can be cumbersome and expensive. In some aspects, methods and compositions are provided herein in which a population of polynucleotides in a sample of fragmented genomic DNA is tagged with n different unique identifiers, wherein n is at least 2 and no more than 100,000*z, wherein z is a measure of central tendency (e.g., mean, median, mode) of an expected number of duplicate molecules having the same start and stop positions. In certain embodiments, n is at least any of 2*z, 3*z, 4*z, 5*z, 6*z, 7*z, 8*z, 9*z, 10*z, 11*z, 12*z, 13*z, 14*z, 15*z, 16*z, 17*z, 18*z, 19*z, 20*z or 100*z (e.g., lower limit). In other embodiments, n is no greater than100,000*z, 10,000*z, 2000*z, 1000*z, 500*z or 100*z (e.g., upper limit). Thus, n can range between any combination of these lower and upper limits. In certain embodiments, n is between 100*z and 1000*z, 5*z and 15*z, between 8*z and 12*z, or about 10*z. For example, a haploid human genome equivalent has about 3 picograms of DNA. A sample of about 1 microgram of DNA contains about 300,000 haploid human genome equivalents. The number n can be between 15 and 45, between 24 and 36, between 64 and 2500, between 625 and 31,000, or about 900 and 4000. Improvements in sequencing can be achieved as long as at least some of the duplicate or cognate polynucleotides bear unique identifiers, that is, bear different tags. However, in certain embodiments, the number of tags used is selected so that there is at least a 95% chance that all duplicate molecules starting at any one position bear unique identifiers. For example, a sample comprising about 10,000 haploid human genome equivalents of cfDNA can be tagged with about 36 unique identifiers. The unique identifiers can comprise six unique DNA barcodes. Attached to both ends of a polynucleotide, 36 possible unique identifiers are produced. Samples tagged in such a way can be those with a range of about 10 ng to any of about 100 ng, about 1 µg, about 10 µg of fragmented polynucleotides, e.g., genomic DNA, e.g., cfDNA.

Accordingly, the present disclosure also provides compositions of tagged polynucleotides. The polynucleotides can comprise fragmented DNA, e.g., cfDNA. A set of polynucleotides in the composition that map to a mappable base position in a genome can be non-uniquely tagged, that is, the number of different identifiers can be at least at least 2 and fewer than the number of polynucleotides that map to the mappable base position. A composition of between about 10 ng to about 10 µg (e.g., any of about 10 ng-1 µg, about 10 ng-100 ng, about 100 ng-10 µg, about 100 ng-1 µg, about 1 µg-10 µg) can bear between any of 2, 5, 10, 50 or 100 to any of 100, 1000, 10,000 or 100,000 different identifiers. For example, between 5 and 100 or between 100 and 4000 different identifiers can be used to tag the polynucleotides in such a composition.

Events in which different molecules mapping to the same coordinate (in this case having the same start/stop positions) and bearing the same, rather than different, tags, are referred to as "molecular collisions". In certain instances, the actual number of molecular collisions may be greater than the number of theoretical collisions, calculated, e.g., as above. This may be a function of uneven distribution of molecules across coordinates, differences in efficiency of ligation between barcodes, and other factors. In this case, empirical methods can be used to determine the number of barcodes needed to approach the theoretical collision number. In one embodiment, provided herein is a method of determining a number of barcodes required to diminish barcode collisions for a given haploid genome equivalent based on length distribution of sequenced molecules and sequence uniformity. The method comprising creating a plurality of pools of nucleic acid molecules; tagging each pool with incrementally increasing numbers of barcodes; and determining an optimal number of barcodes that reduces the number of barcode collisions to a theoretical level, e.g., that could be due to differences in affective barcode concentrations due to differences is pooling and ligation efficiency.

In one embodiment, the number of identifiers necessary to substantially uniquely tag polynucleotides mapping to a region can be determined empirically. For example, a selected number of different identifiers can be attached to molecules in a sample, and the number of different identifiers for molecules mapping to the region can be counted. If an insufficient number of identifiers is used, some polynucleotides mapping to the region will bear the same identifier. In that case, the number identifiers counted will be less than the number of original molecules in the sample. The number of different identifiers used can be iteratively increased for a sample type until no additional identifiers, representing new original molecules, are detected. For example, in a first iteration, five different identifiers may be counted, representing at least five different original molecules. In a second iteration, using more barcodes, seven different identifiers are counted, representing at least seven different original molecules. In a third iteration, using more barcodes, 10 different identifiers are counted, representing at least ten different original molecules. In a fourth iteration, using more barcodes, 10 different identifiers, again, are counted. At this point, adding more barcodes is not likely to increase the number of original molecules detected.

### 5. Sequencing

Sample nucleic acids flanked by adapters with or without prior amplification can be subject to sequencing. Sequencing methods include, for example, Sanger sequencing, high-throughput sequencing, pyrosequencing, sequencing-by-synthesis, single-molecule sequencing, nanopore sequencing, semiconductor sequencing, sequencing-by-ligation, sequencing-by-hybridization, RNA-Seq (Illumina), Digital Gene Expression (Helicos), Next generation sequencing, Single Molecule Sequencing by Synthesis (SMSS) (Helicos), massively-parallel sequencing, Clonal Single Molecule Array (Solexa), shotgun sequencing, Ion Torrent, Oxford Nanopore, Roche Genia, Maxim-Gilbert sequencing, primer walking, sequencing using PacBio, SOLiD, Ion Torrent, or Nanopore platforms. Sequencing reactions can be performed in a variety of sample processing units, which may multiple lanes, multiple channels, multiple wells, or other mean of processing multiple sample sets substantially simultaneously. Sample processing unit can also include multiple sample chambers to enable processing of multiple runs simultaneously.

The sequencing reactions can be performed on one more fragments types known to contain markers of cancer of other disease. The sequencing reactions can also be performed on any nucleic acid fragments present in the sample. The sequence reactions may provide for sequence coverage of the genome of at least 5%, 10%, 15%, 20%, 25%, 30%, 40%, 50%, 60%, 70%, 80%, 90%, 95%, 99%, 99.9% or 100%. In other cases, sequence coverage of the genome may be less than 5%, 10%, 15%, 20%, 25%, 30%, 40%, 50%, 60%, 70%, 80%, 90%, 95%, 99%, 99.9% or 100%.

Simultaneous sequencing reactions may be performed using multiplex sequencing. In some cases, cell-free nucleic acids may be sequenced with at least 1000, 2000, 3000, 4000, 5000, 6000, 7000, 8000, 9000, 10000, 50000, 100,000 sequencing reactions. In other cases, cell-free polynucleotides may be sequenced with less than 1000, 2000, 3000, 4000, 5000, 6000, 7000, 8000, 9000, 10000, 50000, 100,000 sequencing reactions. Sequencing reactions may be performed sequentially or simultaneously. Subsequent data analysis may be performed on all or part of the sequencing reactions. In some cases, data analysis may be performed on at least 1000, 2000, 3000, 4000, 5000, 6000, 7000, 8000, 9000, 10000, 50000, 100,000 sequencing reactions. In other cases, data analysis may be performed on less than 1000, 2000, 3000, 4000, 5000, 6000, 7000, 8000, 9000, 10000, 50000, 100,000 sequencing reactions.

The sequencing method can be massively parallel sequencing, that is, simultaneously (or in rapid succession) sequencing any of at least 100, 1000, 10,000, 100,000, 1 million, 10 million, 100 million, or 1 billion nucleic acid molecules.

### 6. Analysis

Genetic data can be used for characterizing a specific form of cancer. Cancers are often heterogeneous in both composition and staging. Genetic profile data may allow characterization of specific sub-types of cancer that may be important in the diagnosis or treatment of that specific sub-type. This information may also provide a subject or practitioner clues regarding the prognosis of a specific type of cancer and allow either a subject or practitioner to adapt treatment options in accord with the progress of the disease. Some cancers progress, becoming more aggressive and genetically unstable. Other cancers may remain benign, inactive or dormant. The system and methods of this disclosure may be useful in determining disease progression.

The present methods are useful in determining the efficacy of a particular treatment option. The present methods can also be used for detecting genetic variations in conditions other than cancer. Immune cells, such as B cells, may undergo rapid clonal expansion upon the presence certain diseases. Clonal expansions may be monitored using copy number variation detection and certain immune states may be monitored. In this example, copy number variation analysis may be performed over time to produce a profile of how a particular disease may be progressing.

Further, the methods of the disclosure may be used to characterize the heterogeneity of an abnormal condition in a subject, the method comprising generating a genetic profile of extracellular polynucleotides in the subject, wherein the genetic profile comprises a plurality of data resulting from copy number variation and rare mutation analyses. In some cases, including but not limited to cancer, a disease may be heterogeneous. Disease cells may not be identical. In the example of cancer, some tumors are known to comprise different types of tumor cells, some cells in different stages of the cancer. In other examples, heterogeneity may comprise multiple foci of disease. Again, in the example of cancer, there may be multiple tumor foci, perhaps where one or more foci are the result of metastases that have spread from a primary site.

The present methods can be used to generate or profile, fingerprint or set of data that is a summation of genetic information derived from different cells in a heterogeneous disease. This set of data may comprise copy number variation and rare mutation analyses alone or in combination.

The present methods can be used to diagnose, prognose, monitor or observe cancers or other diseases.

### 7. Kits

The disclosure also provides kits for practice of any of the above methods. An exemplary kit includes oligonucleotide probes for targeting and capturing a sequencing panel as discussed herein. Kits can also include packaging, leaflets, CDs or the like providing instructions for practice of the claimed methods.

All patent filings, websites, other publications, accession numbers and the like cited above or below are incorporated by reference in their entirety for all purposes to the same extent as if each individual item were specifically and individually indicated to be so incorporated by reference. If different versions of a sequence are associated with an accession number at different times, the version associated with the accession number at the effective filing date of this application is meant. The effective filing date means the earlier of the actual filing date or filing date of a priority application referring to the accession number if applicable. Likewise, if different versions of a publication, website or the like are published at different times, the version most recently published at the effective filing date of the application is meant unless otherwise indicated. Any feature, step, element, embodiment, or aspect of the invention can be used in combination with any other unless specifically indicated otherwise. Although the present invention has been described in some detail by way of illustration and example for purposes of clarity and understanding, it will be apparent that certain changes and modifications may be practiced within the scope of the appended claims.

### EXAMPLE WORKFLOWS

### EXAMPLE 1: Improving bisulfite sequencing recovery

1) Obtain cfDNA sample.
2) Prepare ends for Y-adapter ligation (T4 DNA ligase): "end-repair" with dmCTP-containing dNTP mix to mark bases synthesized during process (improve methylation calling accuracy), and A-tail. In commercial ssDNA-LP `splint adapter ligation' kits, such as SRSLY from Claret Biosciences, there is a step cocurrent with ligation that phosphorylates 5' ends and optionally removes 3' phosphate groups, preparing them for ligation. This step can be skipped for the hybrid workflow described herein, as it is performed during the upstream 'end-repair' that prepares dsDNA for Y-adapter ligation. In this case 5' hydroxyl group and 3' phosphate group are simplest and most economical modifications to employ. However, if the ssDNA-LP T4 PNK step is left in the protocol, these two modifications cannot be used as they are substrates for T4 PNK activity, which will convert them to ligatable ends. In protocols with a ssDNA-LP PNK step, such suitable modifications to the ssDNA-ends of the Y-adapters are 5' C3 spacer, 5' inverted dideoxy-base, other 5' spacers, 3' C3 spacer, 3' inverted-dT, 3'dideoxy-base, other 3'spacers, as these will all prevent/inhibit T4 PNK activity.
3) Ligate ds-cfDNA molecules with Y-adapters contain methylated cytosines to protect from downstream bisulfite conversion, ssDNA ends in the Y contain specific modifications to prevent ligation through these ends (e.g., 5'OH, 3'P). Y-adapters contain a DNA barcode that serves as molecular barcode and/or to identify molecule as originating in ligation.
4) Post-ligation cleanup. This step is optional, omitting it can potentially improve recovery.
5) Denature DNA and perform bisulfite treatment.
6) Perform ssDNA library prep with splint adapters of two types. (1) double-stranded DNA hybrid with the SP5/read1-primer _sequence and a randomer sequence overhang that is 5' of the reverse-complement of the SP5, (2) double-stranded hybrid with SP7/read2-primer_sequence and a randomer sequence overhang that is 3' of SP7/read2-primer_sequence. Both adapters have modifications at their double-stranded blunt end, such that they cannot be ligated at these ends (e.g., 5'OH, 3'P).
7) Denature to ssDNA and apply single-stranded binding protein (SSB).
8) Ligate splint adapters onto ssDNA not ending with sequences - these are DNA fragment ends missed in first ligation or created by bisulfite-induced fragmentation. Note this ssDNA LP method using splint adapters varies from published protocol/kits that have a phosphorylation step before/concurrent with ligation. This mod hinders recovery in a standard ssDNA LP, but is essential and improves recovery in this approach, in which the Y-adapters are blocked with a 5'OH mod. Alternatively, as discussed above, alternative modifications can be used to block ligation of these ends that are not susceptible to downstream phosphorylation.
9) Post-ligation cleanup. This step is optional, omitting it can potentially improve recovery.
10) Amplify all DNA library molecules that contain either a or splint adapter sequence on both ends. Optionally enrich regions of genome of interest for sequencing through oligo hybrid capture.
11) Sequence all DNA library molecules of interest on NGS platform.
12) Analyze sequencing reads to identify the originating molecule topology and resolve unique molecules from PCR duplicates: (1) "Full length" DNA molecules originating as dsDNA and not fragmented by bisulfite in process as having a barcode on both molecule ends (barcode can be molecular barcode or just ligation identifying barcode). Molecules of these type are resolved through combination of barcodes on ends of molecules and genomic co-ordinates of ends. (2) Partially bisulfite fragmented DNA molecules originating as dsDNA and fragmented by bisulfite as having barcode on one end-only. Molecules of these type are resolved through combination of barcode on one end and genomic co-ordinate of ends. Note that bisulfite fragmentation resulting in one end should increase fragment end diversity which will increase resolution power in using genomic coordinates. (3) Fully bisulfite fragmented DNA molecules originating as dsDNA and fragmented on both ends by bisulfite or originating as ssDNA (minor), which are identified by absence of molecular barcodes on both ends. Molecules are resolved by genomic co-ordinate of ends, which again will have more diversity due to fragmentation.

From multiple NGS reads of the same molecule a consensus sequence is generated that will increase base accuracy, including methylation status.

For "full length" molecules in which both the top and bottom strand have NGS reads, a further consensus can be generated that accounts for dsDNA strand symmetry (in methylation events and somatic mutations).

### EXAMPLE 2: Double-stranded DNA barcode-only ligation

1) End-repair and A-tail (optional) dsDNA.
2) Ligate dsDNA molecules with T-tailed (optional) dsDNA-barcode tags that contain series of non-complementary 5' and 3' overhang sequences that prevent barcodes from ligating to each other/self (similar to 'Y' in Y-adapter). Note that unlike other methods the tag ends should be ligatable in downstream steps. As such the barcode tags may contain 5'P on 3'OH on all ends, not just the desired ligation junction side (T-tail side of barcode). Optionally cleanup first ligation.
3) Subject to ssDNA LP method using splint adapter ligation method as described above with notable difference that a phosphorylation step may be included before/concurrent with the (second) ligation step that may improve recovery here.
4) Amplify all DNA library molecules that contain splint adapter sequence on both ends. Optionally enrich regions of genome of interest for sequencing through oligo hybrid capture.
5) Sequence all DNA library molecules of interest on NGS platform.

6) Analyze sequencing reads to identify the originating molecule topology and resolve unique molecules from PCR duplicates: (2) dsDNA-originating molecules - contain barcode/tag on both ends of the molecule. The DNA barcode combination and genomic co-ordinate of ends can be used to identify molecule. (2) ssDNA-originating molecules - do not contain barcode/tag on either end of the molecule. The genomic coordinates of ends can be used to identify molecule. Note: if molecule has barcode/tag on only one end it is likely a dsDNA molecule underwent partial ligation due to inefficient end-repair, A-tail, first ligation.

### EXAMPLE 3: Hairpin dsDNA-ligation

1) End-repair and A-tail (optional) dsDNA.
2) Ligate dsDNA molecules with T-tailed (optional) with hairpin NGS adapters (such as that used NEBNext kits) modified to have molecular barcodes. Optionally cleanup first ligation.
3) Subject to ssDNA LP method using splint adapter ligation method as described above with notable difference that a phosphorylation step may be included before/concurrent with the (second) ligation step that may improve recovery here.
4) Subject sample to 'User treatment' (NEB; Uracil DNA glycosylase followed by Endonuclease III) to severe hairpin adapted molecules, into linear monomer library molecules easy to amplify.
5) Amplify all DNA library molecules that contain splint adapter sequence on both ends. Optionally - enrich regions of genome of interest for sequencing through oligo hybrid capture.
6) Sequence all DNA library molecules of interest on NGS platform.
7) Identify through sequencing analysis the originating molecule topology and resolve unique molecules from PCR duplicates: (1) dsDNA-originating molecules - contain barcode/tag on both ends of the molecule. The DNA barcode combination and genomic coordinate of ends can be used to identify molecule. (2) ssDNA-originating molecules - do not contain barcode/tag on either end of the molecule. The genomic coordinates of ends can be used to identify molecule. Note: if molecule has barcode/tag on only one end it is likely a dsDNA molecule underwent partial ligation due to inefficient end-repair, A-tail, first ligation.

### EXAMPLE 4: Use of biotinylated Y-adapters to separate ssDNA material

1) End-repair and A-tail (optional) dsDNA.
2) Ligate dsDNA molecules with T-tailed (optional) Y-adapters with molecular barcode with biotin modification (e.g., modified base in oligo). Optionally cleanup first ligation.
3) Subject sample to streptavidin magnetic bead-based separation remove supernatant containing ssDNA molecules to a second reaction vessel. Optional workflow variants include: (1) MSRE treatment applied prior to streptavidin-based separation. Any doubly digested DNA fragments will be retained in ssDNA reaction vessel and potentially converted into libraries. However, these will not contribute noise to methylation measurement as they will not contain molecular barcodes (and treated as fully methylated molecules) and can be potentially identified by fragment end co-ordinates matching MSRE sites. Additionally, if separate enrichments are carried out for ssDNA and post-MSRE dsDNA libraries then the ssDNA enrichment panel can be designed to avoid MSRE fragment information if uninformative. (2) Releasing the dsDNA library from immobilization prior to MSRE treatment. This can be performed by using on the adapters photocleavable biotin, or desbiotin (chased with biotin) or other type biotin base that is cleavable (i.e. biotin-UTP, and applying "user treatment").
4) Subject first reaction vessel containing bead-immobilized dsDNA library molecules to MSRE (methylation sensitive restriction enzyme) treatment to digest library molecules containing unmethylated MSRE sites.
5) Subject second reaction vessel to an ssDNA-LP to prepare contained ssDNA molecules for NGS. First and second reaction vessels can be amplified separately or jointly (with strep beads). Optionally - enrich regions of genome of interest for sequencing through oligo hybrid capture. Note separate enrichment reactions and regions for enrichment can be selected for ssDNA and post-MSRE dsDNA libraries if kept separate in previous step.
6) Sequence all DNA library molecules of interest on NGS platform.
7) Analyze sequencing reads to resolve two types of molecules: (1) "Fully methylated" double stranded DNA originating molecules by presence of molecular barcodes at both ends of molecules and (2) Single stranded DNA(ssDNA) originating molecules by absence of molecular barcodes at both ends of molecules.

Many method variants have been noted with asterisks throughout the specification. Most notably in the splint adapter methods. There are different methods to protect the first ligation (Y-adapter) from re-ligation in the splint adapter step. Additionally, in subsection "Loss of ssDNA molecules and DNA molecule topology information (ssDNA- or dsDNA-originating) in genetic sequencing workflows" it is noted that in the first "dsDNA" ligation step Y-adapters may be used to generate fully formed library molecules at this stage or just dsDNA-identifying tags/barcodes (w/o any end protection) could be ligated on, and the NGS adapters be further appended to these molecules during the ssDNA LP. The former may be advantageous for higher molecular recovery (of the dsDNA molecules), while the latter may be desirable as it employs simpler/cheaper reagents. In the case of the latter, the dsDNA-identifying barcodes can still be molecular barcodes. Furthermore, the first ligation can be performed with hairpin adapters (with molecular/identifying barcodes) that simplifies some aspects of the workflow - not requiring adapter end protection.

It is also possible to use different ssDNA LP methods in place of the "splint adapter" method focused on. Swift/IDT has a ssDNA LP with a first adapter ligation step that method 3' extends ssDNA molecules with a terminal transferase polymerase, which is subsequently/concurrently ligated to splint adapter with an overhang complementary to the extended templates. A primer matching the 3' adapter end is then extended with a polymerase to re-generate a dsDNA molecule that is blunt-ended or A-tailed. Then a second ligation is performed with a blunt or T-tailed adapter. This method is interchangeable for the "splint adapter" method in the "Improve ssDNA recovery and gain molecule topology information (ssDNA vs dsDNA) with 'hybrid' LP workflow." It can also be used in the disclosed method to improve Bisulfite-Seq molecular recovery, albeit only half of the "partially bisulfite fragmented" molecules may be recovered - those that have a bisulfite exposed 3' end.

The disclosed methods can be applied to single-molecule sequencing, which can yield single-site methylation information with added molecule topology information.
The invention is further characterized by the following numbered non-limiting embodiments:
1. A method of preparing a sequencing library from DNA molecules in a sample, the method comprising:
   (a) providing a first population of DNA molecules from the sample, the first population of DNA molecules comprising double-stranded DNA and single-stranded DNA;
   (b) ligating a first set of adapters comprising molecular barcodes configured to attach to a plurality of the double-stranded DNA molecules to generate a second population comprising a plurality of adapter-ligated double-stranded DNA molecules, wherein the adapters are ligated to both or one end of the double-stranded DNA molecules, and a plurality of unligated DNA molecules;
   (c) subjecting the second population to a treatment that denatures and fragments a plurality of the adapter ligated DNA molecules and the unligated DNA molecules to generate a third population of DNA molecules comprising single-stranded DNA molecules having adapter at both, one, and/or neither ends and fragmented single-stranded DNA molecules, wherein the fragmented single-stranded DNA molecules comprise fragments having one and/or no adapter ligated to an end of the fragment; and
   (d) ligating a second set of adapters to a subset of molecules in the third population which either have an adapter or no adapter ligated to the fragment, thereby generating tagged DNA molecules comprising at least two of:
      (i) single-stranded adapter-ligated DNA comprising adapters from the first set of adapters ligated to both ends of the molecule,
      (ii) single-stranded adapter-ligated DNA comprising one adapter from the first set of adapters ligated to one end of the molecule and one adapter from the second set of adapters ligated to the other end of the molecule, and
      (iii) single-stranded adapter-ligated DNA comprising adapters from the second set of adapters ligated to both ends of the molecule,
   thereby providing a sequencing library from the population of DNA molecules in the sample.
2. The method of embodiment 1, wherein the first population of DNA molecules comprises double-stranded and single-stranded cell-free DNA (cfDNA).
3. The method of embodiment 1 to 2, wherein the first set of adapters are Y-shaped adapters
4. The method of embodiments 1 to 3, wherein the first set of adapters are protected from the treatment in (c).
5. The method of any one of embodiments 1 to 4, wherein the first set of adapters further comprise single-stranded ends that are protected from ligation using modifications comprising 5'OH and/or 3'P.
6. The method of embodiments 1 to 4, wherein the first set of adapters comprise single-stranded ends that are protected from ligation not using modifications comprising 5'OH and/or 3'P when T4 PNK is used in (d).
7. The method of embodiment 1 to 4, wherein the first set of adapters further comprise single-stranded ends that are protected from ligation using modifications comprising 5' C3 spacer, 5' inverted dideoxy-base, other 5' spacers, 3' C3 spacer, 3' inverted-dT, 3'dideoxy-base, other 3'spacers, when T4 PNK is used in(d).
8. The method of any one of embodiments 1 to 7, wherein the first set of adapters comprise universal amplification sequences.
9. The method of any one of embodiments 1 to 8, wherein the molecular barcode differentiates molecules ligated in (b) from molecules ligated in (d).
10. The method of any one of embodiments 1-4 or 6-9, wherein T4 PNK is used to phosphorylate the population of DNA molecules prior to ligation in (b).
11. The method of any one of embodiments 1-4 or 6-10, wherein T4 PNK is used to phosphorylate the population of DNA molecules prior to ligation in (d).
12. The method of any one of embodiment 1 to 11, wherein the treatment that denatures and fragments the second population of DNA molecules comprises at least one of : bisulfite conversion, Tet-assisted bisulfite conversion, Tet-assisted conversion with a substituted borane reducing agent, optionally wherein the substituted borane reducing agent is 2-picoline borane, borane pyridine, tert-butylamine borane, or ammonia borane.
13. The method of any one of embodiments 1 to 12, wherein the treatment that denatures and fragments the second population of DNA molecules comprises chemical-assisted conversion with a substituted borane reducing agent, optionally wherein the substituted borane reducing agent is 2-picoline borane, borane pyridine, tert-butylamine borane, or ammonia borane.
14. The method of any one of embodiments 1 to 13, wherein the first set of adapters comprise methylated cytosines to protect them from the treatment.
15. The method of any one of embodiments 1 to 14, wherein the second set of adapters are `splint' adapters that contain a 5' or 3' overhangs.
16. Then method of any one of embodiments 1 to 15, wherein the second set of adapters comprise universal amplification sequences.
17. The method of any one of embodiments 1 to 16, wherein the second set of adapters comprise (i) adapters with a double stranded portion and a single stranded overhang comprising a randomer sequence that is 5' of the reverse strand and (ii) adapters with a double stranded portion and a single stranded overhang comprising a randomer sequence that is 3' of the reverse strand.
18. Then method of any one of embodiments 1 to 17, wherein the second set of adapters selectively tag only DNA molecule ends lacking the first adapter sequence.
19. The method of any one of the preceding embodiments, further comprising amplifying the molecules in (d) (i)-(iii) to generate duplicated DNA molecules.
20. The method of embodiment 19, further comprising sequencing the amplified DNA molecules to generate sequencing reads.
21. The method of embodiment 20, wherein prior to sequencing, the amplified molecules are captured to enrich one or more target regions.
22. The method of embodiment 21, wherein the sequencing reads are analyzed to resolve unique molecules from PCR duplicates by identify molecules comprising the same end co-ordinates and/or molecular barcodes.
23. The method of any one of embodiments 1 to 22, wherein the DNA molecules that have the first adapter at one end and the second adapter at the other end will have greater diversity in the end-coordinates than molecules in (d)(i), wherein said diversity in fragment ends improves accuracy to resolve unique molecules from PCR duplicates.
24. The method of any one of embodiments 1 to 23, wherein the DNA molecules that have the second adapter at both ends in (d)(iii) will have greater diversity in the end-coordinates than molecules in (d)(i) and (ii), wherein said diversity improves accuracy to resolve unique molecules from PCR duplicates.
25. The method of any one of embodiments 1 to 24, wherein the end-coordinate diversity improves accuracy to resolve unique molecules from PCR duplicates.
26. The method of any one of embodiments 1 to 25, wherein the end-coordinates and molecular barcodes improves accuracy to resolve unique molecules from PCR duplicates.
27. The method of any one of embodiments 1 to 26, wherein the forward and reverse strand symmetry improves accuracy to detect methylation status.
28. The method of any one of embodiments 1 to 27, wherein the forward and reverse strand symmetry improves accuracy to detect somatic mutations.
29. The method of any one of the preceding embodiments, wherein for DNA molecules that have the first adapter at both ends in (d)(i), a consensus is generated that accounts for forward and reverse strand symmetry to determine methylation status and/or somatic mutations.
30. The method of any of the preceding embodiments, wherein prior to (b), the DNA molecules in the first population are end-repaired and/or A-tailed.
31. A method of analyzing a population of DNA molecules in a sample, the method comprising:
   (a) ligating a first set of adapters comprising molecular barcodes to at least a subset of DNA molecules within the population of DNA molecules,
   wherein the adapters are ligated at both ends of the DNA molecules to generate adapter ligated DNA molecules;
   (b) subjecting the population of DNA molecules to a biochemical treatment that denatures and randomly fragments a plurality of the DNA molecules, thereby producing fragmented DNA molecules that have =<2 first adapter on their ends;
   (c) ligating a second set of adapters to the DNA molecules in the population that have <2 first adapter sequences on their ends to generate a population of tagged DNA molecules comprising at least two of:
      (i) DNA molecules that have the first adapter at both ends,
      (ii) DNA molecules that have the first adapter at one end and the second adapter at the other end,
      (iii) DNA molecules that have the second adapter at both ends;
   (d) sequencing the population of tagged DNA molecules from (c) to generate sequencing reads; and
   (e) analyzing the sequencing reads to detect parallel signals associated with disease.
32. The method of embodiment 31, wherein the signals associated with disease further comprise one or more of : short to long cfDNA fragment ratio, differential chromatin architecture, nucleosome structure, epigenetic, and/or genetic information.
33. A method of preparing a sequencing library from a population of DNA molecules in a sample, the method comprising:
   (a) ligating a pair of molecular barcodes to a subset of DNA molecules in the population, wherein the pair of molecular barcodes further comprise (i) a first set of molecular barcodes comprising a ligatable end and a 3' single-stranded overhang at the opposite end and (ii) a second set of molecular barcodes comprising a ligatable end and a 5' single-stranded overhang at the opposite end, to generate a plurality of tagged DNA molecules having a molecular barcode at each end; and
   (b) ligating a set of adapters to a plurality of the tagged DNA molecules and a plurality of non-tagged DNA molecules that did not undergo ligation in (a), to generate (i) adapter-tagged molecules comprising molecular barcodes and (ii) adapter tagged molecules that do not contain the barcode,
   thereby providing the sequencing library from the population of DNA molecules in the sample.
34. The method of embodiment 33, wherein the 5' and 3' overhang sequences prevent the pair of barcodes from ligating to each other or self-ligate.
35. The method of embodiment 33 or 34, wherein the ligatible end comprises a T tail or an A tail.
36. The method of any one of embodiments 33 to 35, wherein the ligatible end comprises a blunt end.
37. The method of any one of embodiments 33 to 36, wherein the barcode further comprises an adapter sequence.
38. The method of any one of embodiments 33 to 37, wherein the adapters are attached to the 3' and 5' single-stranded overhangs of the tagged DNA molecules.
39. The method of any one of embodiments 33 to 38, wherein the set of adapters comprise (i) adapters with a double stranded portion and a single stranded overhang comprising a randomer sequence that is 5' of the reverse strand and (ii) adapters with a double stranded portion and a single stranded overhang comprising a randomer sequence that is 3' of the reverse strand.
40. The method of any one of embodiments 33 to 39, wherein the adapters are `splint' adapters that contain a 5' or 3' overhangs.
41. The method of any one of embodiments 33 to 40, wherein the adapters comprise universal amplification sequences.
42. The method of any one of embodiments 33 to 41, further comprising, amplifying the sequencing library to generate amplified (i) adapter-tagged molecules comprising molecular barcodes and (ii) adapter tagged molecules that do not contain the barcode.
43. The method of any one of embodiments 33 or 42, further comprising selectively enriching the library to isolate a subset of the molecules in b(i)(ii).
44. The method of any one of embodiments 33 to 43, further comprising sequencing the library of molecules in b(i)(ii) to generate sequencing reads.
45. The method of embodiment 44, wherein the selective enrichment is performed by hybridization or amplification techniques.
46. The method of any one of embodiments 43 to 45, wherein the enriched subset of the molecules in b(i)(ii) are associated with a disease.
47. The method of embodiment 46, wherein the disease is cancer, Alzheimer's, hypertriglyceridemia, coronary artery disease.
48. A method of preparing a sequencing library from a population of DNA molecules in a sample, the method comprising:
   (a) ligating a first set of adapters comprising a capture-label to at least a subset of DNA molecules to generate a first subset of adapter-ligated DNA molecules comprising a capture-label, wherein the adapters are ligated at both ends of the DNA molecules;
   (b) separating the adapter ligated DNA molecules comprising the capture-label by contacting the population of DNA molecules with a capture molecule to generate, (i) a first subset of adapter-ligated DNA molecules comprising a capture-label, wherein the label is bound to the capture molecule, (ii) non-adapted DNA molecules; and
   (c) ligating a second set of adapters to at least a subset of the non-adapted DNA molecules to generate a second subset of adapter-ligated DNA molecules, wherein the adapters are ligated at both ends of the DNA molecules,
   thereby providing a sequencing library from the populating from DNA molecules in the sample.
49. The method of embodiment 48, wherein the first set of adapters further comprise y-shape adapters.
50. The method of any one of embodiments 48 to 49, wherein first set of adapters further comprise a molecular barcode.
51. The method of any one of embodiments 48 to 50, wherein the capture-label comprises an affinity ligand.
52. The method of any one of embodiments 51, wherein the affinity ligand is biotin or photocleavable biotin.
53. The method of embodiment 52, wherein the photocleavable biotin is biotin-UTP.
54. The method of any one of embodiments 48 to 53, wherein the separating in (b) further comprises affinity purification using at least one capture molecule.
55. The method of any one of embodiments 48 to 54, wherein the capture molecule is streptavidin or magnetic beads coated with streptavidin.
56. The method of any one of embodiments 48 to 55, wherein the first set of adapter ligated molecules are subjected to a treatment that digests unmethylated DNA.
57. The method of any one of embodiments 48 to 56, wherein the second set of adapters are `splint' adapters that contain a 5' or 3' overhangs.
58. The method of embodiments 48 to 57, wherein the second set of adapters comprise universal amplification sequences.
59. The method of embodiments 48 to 58, wherein the second set of adapters comprise (i) adapters with a double stranded portion and a single stranded overhang comprising a randomer sequence that is 5' of the reverse strand and (ii) adapters with a double stranded portion and a single stranded overhang comprising a randomer sequence that is 3' of the reverse strand.
60. The method of any one of embodiments 48 to 59, further comprising sequencing the library to generate sequencing reads.
61. The method of embodiment 60, further 'comprising amplifying the library prior to sequencing.
62. The method of embodiment 61, further comprising selectively enriching the library prior to sequencing.
63. The method of any one of embodiments 60 to 62, further comprising analyzing the sequencing reads to determine the methylation status at one or more genetic loci.
64. The method of any one of embodiments 60 to 62, further comprising analyzing the sequencing reads to determine the molecular topology (e.g., double-stranded originating molecules and single-stranded originating molecules) of the second subset of adapter-ligated DNA molecules in(c).
65. The method of any one of embodiments 60 to 64, further comprising analyzing the sequencing reads to detect parallel signals associated with disease.
66. The method of any one of embodiments 61 to 65, wherein the disease comprises cancer, Alzheimer's, hypertriglyceridemia, or coronary artery disease.

## Claims

1. A method of preparing a sequencing library from a population of DNA molecules in a sample, the method comprising:
(a) ligating a first set of adapters comprising a capture-label to at least a subset of DNA molecules to generate a first subset of adapter-ligated DNA molecules comprising a capture-label, wherein the adapters are ligated at both ends of the DNA molecules;
(b) separating the adapter ligated DNA molecules comprising the capture-label by contacting the population of DNA molecules with a capture molecule to generate, (i) a first subset of adapter-ligated DNA molecules comprising a capture-label, wherein the label is bound to the capture molecule, (ii) non-adapted DNA molecules; and
(c) ligating a second set of adapters to at least a subset of the non-adapted DNA molecules to generate a second subset of adapter-ligated DNA molecules, wherein the adapters are ligated at both ends of the DNA molecules,
thereby providing a sequencing library from the populating from DNA molecules in the sample.

2. The method of claim 1, wherein the first set of adapters further comprise y-shape adapters.

3. The method of claim 1 or claim 2, wherein first set of adapters further comprise a molecular barcode.

4. The method of any one of claims 1 to 3, wherein the capture-label comprises an affinity ligand, optionally wherein the affinity ligand is biotin or photocleavable biotin, further optionally wherein the photocleavable biotin is biotin-UTP.

5. The method of any one of claims 1 to 4, wherein the separating in (b) further comprises affinity purification using at least one capture molecule.

6. The method of any one of claims 1 to 5, wherein the capture molecule is streptavidin or magnetic beads coated with streptavidin.

7. The method of any one of claims 1 to 6, wherein the first set of adapter ligated molecules are subjected to a treatment that digests unmethylated DNA.

8. The method of any one of claims 1 to 7, wherein the second set of adapters are `splint' adapters that contain a 5' or 3' overhangs.

9. The method of claims 1 to 8, wherein the second set of adapters comprise universal amplification sequences.

10. The method of claims 1 to 9, wherein the second set of adapters comprise (i) adapters with a double stranded portion and a single stranded overhang comprising a randomer sequence that is 5' of the reverse strand and (ii) adapters with a double stranded portion and a single stranded overhang comprising a randomer sequence that is 3' of the reverse strand.

11. The method of any one of claims 1 to 10, further comprising sequencing the library to generate sequencing reads.

12. The method of claim 11, further comprising amplifying the library prior to sequencing, and optionally further comprising selectively enriching the library prior to sequencing.

13. The method of claim 11 or claim 12, further comprising analyzing the sequencing reads to determine
(i) the methylation status at one or more genetic loci; or
(ii) the molecular topology, e.g., double-stranded originating molecules and single-stranded originating molecules, of the second subset of adapter-ligated DNA molecules in (c).

14. The method of any one of claims 11 to 13, further comprising analyzing the sequencing reads to detect parallel signals associated with disease, optionally wherein the disease comprises cancer, Alzheimer's, hypertriglyceridemia, or coronary artery disease.

15. Use of the method of any one of the preceding claims to generate a profile, fingerprint, or set of data that is a summation of genetic information derived from different cells in a heterogeneous disease, for example wherein the set of data comprises copy number variation and rare mutation analyses alone or in combination.
